# EUROPEAN PATENT SPECIFICATION

(11) **EP 3 687 516 B1**
(45) Date of publication and mention of the grant of the patent: **30.09.2026**
(21) Application number: 18811676.8
(22) Date of filing: 25.09.2018
(51) Int. Cl.: A61K 31/137, A61K 45/06, A61P 25/00, A61P 25/28

(54) **IMPROVEMENT IN COGNITIVE FUNCTION IN PATIENTS WITH DRAVET SYNDROME OR LENNOX-GASTUAT SYNDROME WITH FENFLURAMINE**
VERBESSERUNG DER KOGNITIVEN FUNKTION BEI PATIENTEN MIT DEM DRAVET-SYNDROM ODER LENNOX-GASTUAT-SYNDROM MIT FENFLURAMIN
AMÉLIORATION DE LA FONCTION COGNITIVE CHEZ DES PATIENTS ATTEINTS DU SYNDROME DE DROVELT OU DU SYNDROME DE LENOX-GASTUAT AVEC DE LA FENFLURAMINE

(30) Priority: 26.09.2017 US 201762563255 P; 27.09.2017 US 201762564225 P; 31.10.2017 US 201762579450 P; 30.11.2017 US 201762593029 P; 07.02.2018 US 201862627329 P; 19.04.2018 US 201862660145 P; 10.05.2018 US 201862669833 P; 11.07.2018 US 201862696801 P; 24.09.2018 US 201816140312
(43) Date of publication of application: 05.08.2020
(73) Proprietor: Zogenix International Limited, Windlesham, Surrey GU20 6PP (GB)
(72) Inventor: MORRISON, Glenn, Emeryville, California 94608 (US); GAMMAITONI, Arnold, Emeryville, California 94608 (US); BOYD, Brooks M., Emeryville, California 94608 (US)
(74) Representative: Heller, Benjamin Henry
(86) International application number: PCT/US2018/052596
(87) International publication number: WO 2019/067419

(56) References cited:
- WO-A1-2018/037306
- US-A1- 2017 056 344
- US-A1- 2017 071 949
- M. BOEL ET AL: "Add-On Therapy of Fenfluramine in Intractable Self-Induced Epilepsy", NEUROPEDIATRICS, vol. 27, no. 04, 1 August 1996 (1996-08-01), DE, pages 171 - 173, XP055565166, ISSN: 0174-304X, DOI: 10.1055/s-2007-973781
- ADRIENNE VIOLA ET AL: "The Behavior Rating Inventory of Executive Function (BRIEF) to Identify Pediatric Acute Lymphoblastic Leukemia (ALL) Survivors At Risk for Neurocognitive Impairment :", JOURNAL OF PEDIATRIC HEMATOLOGY/ONCOLOGY, vol. 39, no. 3, 1 April 2017 (2017-04-01), US, pages 174 - 178, XP055566595, ISSN: 1077-4114, DOI: 10.1097/MPH.0000000000000761
- C. B. CATARINO ET AL: "Dravet syndrome as epileptic encephalopathy: evidence from long-term course and neuropathology", BRAIN., vol. 134, no. 10, 29 June 2011 (2011-06-29), GB, pages 2982 - 3010, XP055566747, ISSN: 0006-8950, DOI: 10.1093/brain/awr129
- ANDREAS BRUNKLAUS ET AL: "Dravet syndrome-From epileptic encephalopathy to channelopathy", EPILEPSIA, vol. 55, no. 7, 16 May 2014 (2014-05-16), NEW YORK, US, pages 979 - 984, XP055566750, ISSN: 0013-9580, DOI: 10.1111/epi.12652

## Description

### FIELD OF THE INVENTION

This invention relates generally to the field of improving cognitive function. Cognitive function includes processes such as thinking and language development and language use, visual-spatial perception, and memory abilities, as well as anticipation, planning, judgement, self-awareness, executive function and decision making. Specifically, methods are provided for improving cognitive function by treating the patient with fenfluramine or a pharmaceutically acceptable salt thereof. Improvement in cognitive function may be demonstrated by measuring changes in any one of various scales, such as, for example, by obtaining a baseline measurement of function. Cognitive function may be measured using the Behavior Rating Inventory of Executive Function (BRIEF), the Wechsler memory scale, the MOCA (Montreal Cognitive Assessment) scale, the Executive Abilities: Measures and Instruments for Neurobehavioral Evaluation and Research (EXAMINER), and central nervous system (CNS) functioning of the patient may be measured using a Clinical Global Impression (CGI) scale or other validated scale measuring cognitive and other neurologic function as a pre-treatment test score, and after treatment with fenfluramine, the test can be re-administered to obtain a post-treatment cognitive test score or rating. Thus, an improvement in cognitive and other aspects of CNS function, as measured by an improvement in, for example, a BRIEF score or a CGI score, can be observed and quantitated. In some embodiments, the patient is also being treated for the symptoms of epilepsy.

### BACKGROUND

Fenfluramine, i.e. 3-trifluoromethyl-N-ethylamphetamine is an amphetamine derivative having the structure:
Structure 1

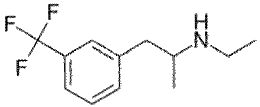

### Systematic (IUPAC) name (RS)-N-ethyl- 1-[3-(trifluoromethyl)phenyl]propan- 2-amine

Fenfluramine was first marketed in the US in 1973 to treat obesity. However, in 1997, it was withdrawn from the US and global market as its use was associated with the onset of cardiac valvulopathy and pulmonary hypertension. Subsequently, the drug was withdrawn from sale globally and is no longer indicated for use in any therapeutic area.

Without being bound by theory, the adverse effects associated with the use of fenfluramine as an anorexic agent are thought to be attributable to the interaction of fenfluramine's major metabolite norfenfluramine with the 5-HT₂B receptor, which has been reported to be associated with cardiac valvulopathy. Fenfluramine is metabolized *in vivo* into norfenfluramine by cytochrome P450 enzymes in the liver. Cytochrome P450 enzymes such as CYP2D6, CYP2B6 and CYP1A2 are primarily responsible for the production of norfenfluramine from fenfluramine in humans. The enzymes CYP2C9, CYP2C19 and CYP3A4 are also involved. Such metabolism includes cleavage of an N-ethyl group to produce norfenfluramine as shown below.

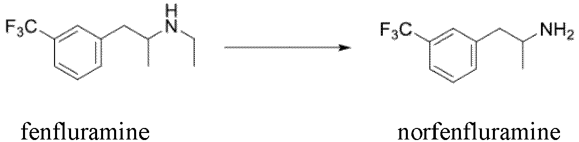

Fenfluramine acts primarily as a serotonin releasing agent. Serotonin (also known as "5-hydroxytryptamine" or "5-HT") is a neurotransmitter that is believed to modulate numerous sensory, motor and behavioral processes in the mammalian nervous system. These diverse responses are elicited through the activation of a large family of receptor subtypes.

Fenfluramine and its major metabolite, norfenfluramine, were reported to be potent substrates for norepinephrine transporters. (Rothman, et al., J. Pharmacol. Exp. Ther. 305(3):1191-9). Fenfluramine causes the release of serotonin by disrupting vesicular storage of the neurotransmitter, and reversing serotonin transporter function. Fenfluramine also acts as a norepinephrine releasing agent to a lesser extent, particularly via its active metabolite norfenfluramine. In addition to monoamine release, while fenfluramine binds only very weakly to the serotonin 5-HT₂ receptors, norfenfluramine binds to and activates the serotonin 5-HT₂B and 5-HT₂C receptors with high affinity and the serotonin 5-HT₂A receptor with moderate affinity. The result of the increased serotonergic and noradrenergic neurotransmission is a feeling of fullness and reduced appetite. Thus, in subjects treated with fenfluramine, weight loss, anorexia and/ or wasting may be observed.

Despite past cardiovascular safety concerns that arose when high doses of fenfluramine were used for treatment of adult obesity, attempts have been made to identify further therapeutic uses for that product, while weighing the known cardiovascular risks of fenfluramine against potential therapeutic benefits. One disorder for which new treatment options are sorely needed is epilepsy, and in particular, epilepsy syndromes which are refractory to known treatments. Epilepsy is a functional disturbance of the central nervous system (CNS) induced by abnormal electrical discharges and marked by a susceptibility to recurrent seizures. There are numerous causes of epilepsy including, but not limited to birth trauma, perinatal infection, anoxia, infectious diseases, ingestion of toxins, tumors of the brain, inherited disorders or degenerative disease, head injury or trauma, metabolic disorders, cerebrovascular accident and alcohol withdrawal.

A large number of compounds may be used to treat different types of epilepsy, and different epilepsy subtypes respond differently to different anticonvulsant drugs. For example, cannabidiol has been studied for treatment of drug-resistant seizures in Dravet syndrome and was reported to reduce convulsive-seizure frequency (Devinsky, et al., 2017, New Engl. J. Med. 376(21):2011-2020).

Dravet syndrome (DS) is a devastating genetic epileptic encephalopathy of infantile onset, frequently caused by mutations or deletions in a neuronal voltage-gated sodium channel (SCN1A). Initially, in the first year of life, the patient with DS experiences prolonged seizures, and in their second year, additional types of seizure begin to occur, typically coinciding with a developmental decline, possibly due to repeated seizures causing brain damage such as cerebral hypoxia. Eventually, this form of pediatric epilepsy leads to poor and/or delayed development of language, disruption of autonomic function, and motor and cognitive/intellectual and behavior impairments. Children with Dravet syndrome are likely to experience multiple seizures per day, and have a higher risk of sudden unexplained death in epilepsy and episodes of uncontrolled status epilepticus. Seizure management includes treatment with benzodiazepines, valproate, and/or stiripentol. Some reduction in seizure activity has been reported with the use of bromides and topiramate, or a ketogenic diet. Despite these options, available antiepileptic drugs (AEDs) do not achieve adequate seizure control in most DS patients.

While a particular drug may be effective against one form of epilepsy, it may be wholly ineffective against others, or even contra-indicated due to exacerbation of symptoms, such as worsening the frequency and severity of the seizures. As a result, efficacy of a particular drug with respect to a particular type of epilepsy is wholly unpredictable, and the discovery that a particular drug is effective in treating a type of epilepsy for which that drug was not previously known to be effective is nearly always surprising, even in cases where the drug is known to be effective against another epilepsy type. Furthermore, treatment of epilepsy with fenfluramine can contra-indicate co-administration of and/or treatment with other therapeutic agents.

Children and adults suffering from epileptic encephalopathies such as Dravet syndrome and Lennox-Gastaut syndrome often experience comorbid impairment of cognitive function, including such abilities as self-regulation, inhibition of impulses and/or attentional control, emotional control, problem solving, tolerating change and/or switching attention, initiating / generating ideas, working memory, planning and organization, *etc.* Cognitive function is sometimes assessed using lab-based performance measures, or using tests such as the Behavior Rating Inventory of Executive Function (BRIEF), the Wechsler memory scale, the MOCA (Montreal Cognitive Assessment) scale, the Executive Abilities: Measures and Instruments for Neurobehavioral Evaluation and Research (EXAMINER), or other validated scale measuring cognitive function.

A more general rating scale for mental and psychiatric conditions is the Clinical Global Impression, CGI, developed for use in NIMH-sponsored clinical trials to provide a brief, stand-alone assessment of the clinician's view of the patient's global functioning prior to and after initiating a study medication. The CGI provides an overall clinician-determined summary measure that takes into account all available information, including a knowledge of the patient's history, psychosocial circumstances, symptoms, behavior, and the impact of the symptoms on the patient's ability to function.

The CGI actually comprises two companion one-item measures evaluating the following: (a) severity of symptoms from 1 to 7 which establishes a baseline for comparison and (b) change from the initiation of treatment on a similar seven-point scale. Subsequent to a clinical evaluation, the CGI form can be completed in less than a minute by an experienced rater. In practice, the CGI captures clinical impressions which encompass more than symptom checklists. It is readily understandable and can be used with relative ease by the non-researcher clinician. Beyond that, the CGI can track clinical progress across time and has been shown to correlate with longer, more time-consuming rating instruments across a wide range of psychiatric and central nervous system dysfynctions.

In clinical practice, the CGI is administered by an experienced clinician who is familiar with the disease under study and the likely progression of treatment. Consequently, the CGI rater can make an expert clinical global judgment about the severity of the illness across various time points within the context of that clinical experience. The clinician makes a judgment about the total picture of the patient at each visit: the illness severity, the patient's level of distress and other aspects of impairment, and the impact of the illness on functioning. The CGI is rated without regard to the clinician's belief that any clinical changes are or are not due to medication and without consideration of the etiology of the symptoms.

The CGI-I rating may also be made by a parent or caregiver or anyone who observes and interacts with the patient on a frequent basis. Ratings by the parent/caregiver are not based on clinical experience with other patients having the disease but are made on more frequent observation of the subject which may identify fluctuations in the individual patient's mental and psychiatric functioning.

There is a long-felt need to provide methods and compositions for improving patients' cognitive function, particularly in children and young adults. In some embodiments, the patient is also being treated for an epileptic disease or disorder, e.g., Dravet Syndrome and/or Lennox-Gastaut syndrome. In some embodiments of this method, fenfluramine is the only pharmaceutically active ingredient administered to the patient. In some embodiments of this method, fenfluramine is used as an adjunctive therapy in a patient. The present disclosure helps in meeting that need, as it relates to the unexpected and surprising discovery that administration of fenfluramine over a period of time is associated with an improvement on at least one scale that measures cognitive function, such as, for example, BRIEF score. As set forth herein, it has been discovered that administration of fenfluramine can be beneficial in treating diseases and disorders affecting cognitive function.

### Cited documents

US2017/071949A1 describes formulations for and methods of treatment of Dravet syndrome that avoid side effects.
US2017/056344A1 describes a method of treating and/or preventing symptoms of Lennox-Gastaut Syndrome (LGS) in a patient such as a patient previously diagnosed with Lennox Syndrome, by administering an effective dose of fenfluramine or its pharmaceutically acceptable salt to that patient.
Boel and Casaer, 1996, Neuropediatrics. 27(4):171-3. doi: 10.1055/s-2007-973781 describes administration of fenfluramine as an add-on therapy to eleven institutionalized children (7 girls, 4 boys) with refractory epilepsy.
Viola, et al, 2017, J Pediatr Hematol Oncol. 39(3): 174-178. doi: 10.1097/MPH.0000000000000761 describes a study to determine if the Behavior Rating Inventory of Executive Function (BRIEF) Parent Form identified leukemia survivors with cognitive impairment.
Catarino, et al, 2011, Brain, 134(10):2982-3010. doi:10.1093/brain/awr129 describes performance of a systematic neuropathology study, with post-mortem material from three adult cases with Dravet syndrome, in comparison with controls and a range of relevant paediatric tissue.
Brunklaus and Zuberi, 2014, Epilepsia.55(7):979-84. doi: 10.1111/epi.12652 describes a review of recent evidence relating to the pathogenicity of SCN1A mutations in Dravet syndrome and the effect these have on the wider disease phenotype and discusses whether knowledge of specific genotypes can influence clinical practice.

### SUMMARY OF THE INVENTION

The present invention is defined in the appended claims. According to a first aspect of the present invention, herein provided is a formulation for use in a method of improving cognitive function in a patient with Dravet syndrome or Lennox-Gastaut syndrome (as measured by a test such as, for example the Behavior Rating Inventory of Executive Function (BRIEF), the Wechsler memory scale, the MOCA (Montreal Cognitive Assessment) scale, the Executive Abilities: Measures and Instruments for Neurobehavioral Evaluation and Research (EXAMINER), or other validated scale measuring cognitive function), wherein the formulation comprises a therapeutically effective dose of fenfluramine, or a pharmaceutically acceptable salt thereof. In some embodiments, the BRIEF test is administered to the patient before and after treatment with fenfluramine to assess cognitive function and measure / quantify improvement. In some embodiments, the fenfluramine is administered for a period of months or years (e.g., one, two three, 6, 9, 12, 15, 18, 21, etc. months, up to an including three years, for example), before measuring/observing a change in cognitive function.

Also herein provided is a formulation for use in a method of improving cognitive function, in a patient with Dravet syndrome or Lennox-Gastaut syndrome as measured, for example, by the Clinical Global Impression scale, such as the Clinical Global Impression of Improvement (CGI-I) wherein the formulation comprises a therapeutically effective dose of fenfluramine, or a pharmaceutically acceptable salt thereof. In some embodiments, the CGI-I is rated by the treating clinician before and after treatment with fenfluramine to assess global mental and psychiatric function and measure / quantify improvement. In another embodiment ratings are made by a parent or caregiver. In some embodiments, the fenfluramine is administered for a period of weeks, months or years (e.g., one, two three, 6, 9, 12, 15, 18, 21, etc. months, up to an including 3 years, for example), before measuring/observing a change in function. In some embodiments improvements in the patient's CGI-I rating continue to improve over a period of months or years.

.

According to a further aspect of the present invention, the patient may also be, have been, or is being treated for epilepsy. According to a further aspect of the present invention, the patient diagnosed with epilepsy is 18 years of age or younger. According to a further aspect of the present invention, the patient diagnosed with epilepsy is an adult over 18 years of age.

According to a further aspect of the present invention, the fenfluramine may be administered in a dosage form selected from the group consisting of oral, injectable, transdermal, inhaled, nasal, buccal, rectal, vaginal and parenteral delivery.

According to a further aspect of the present invention, the dosage form is an oral composition in an amount selected from the group consisting of 30 mg/day or less, 20 mg/day or less, 10 mg/day or less and 5 mg/day or less.

According to a further aspect of the present invention, at least one co-therapeutic agent also may be co-administered to the patient/subject, wherein the agent is selected from the group consisting of Brivaracetam, bromides (e.g., Potassium Bromide, Sodium Bromide), Cannabidiol, Carbamazepine, Clonidine, Ergenyl Chrono, Ethosuximide, Felbamate, Fosphenytoin, Lacosamide, Lamotrigine, Levetiracetam, Levocarnitine, Mesuximide, Nitrazepam, Oxcarbazepine, Perampanel, Phenobarbital, Pregabalin, Progabide, Pyridoxine, Rufinamide, Stiripentol, Sultiame, Tizanidine, Topiramate, Valproate semisodium, Valproate sodium, Valproic acid, Verapamil, Zonisamide, and benzodiazepines such as Clobazam, Clonazepam, Diazepam, Ethyl Loflazepate, Lorazepam and Midazolam, and a pharmaceutically acceptable salt or base of any of these.

According to a further aspect, the subject/patient may have been previously treated with a medication, prior to treatment with fenfluramine, wherein the agent is selected from Acetazolamide, Brivaracetam, Carbamazepine, Clobazam, Clonazepam, Diazepam, Ergenyl Chrono, Ethosuximide, Felbamate, Gabapentin, Lacosamide, Lamotrigine, Levetiracetam, Lorazepam, Mesuximide, Oxcarbazepine, Perampanel, Phenobarbital, Phenytoin, Phenytoin sodium, Pregabalin, Rufinamide, Stiripentol, Sultiame, Topiramate, Valproate semisodium, Valproate sodium, Valproic acid, Vigabatrin, Zonisamide, and a pharmaceutically acceptable salt or base of any of these.

According to a further aspect of the present invention, the fenfluramine treatment continues over a period of time and in amounts effective to improve the cognitive function, which can be assessed via improvements on validated scales, such as, but not limited to, the Behavior Rating Inventory of Executive Function (BRIEF), the Wechsler memory scale, the MOCA (Montreal Cognitive Assessment) scale, the Executive Abilities: Measures and Instruments for Neurobehavioral Evaluation and Research (EXAMINER), or other validated clinical and/or index scales which measure cognitive function.

Within the BRIEF test, there are several scales which can be used to measure specific aspects of cognitive function. The BRIEF has two main indices: the Behavioral Regulation Index (BRI) (including the Inhibit, Shift, and Emotional Control scales) and the Metacognition Index (MI) (including the Initiate, Working Memory, Plan/Organize, Organization of Materials, and Monitor scales).

In some embodiments of this method, fenfluramine is the only pharmaceutically active ingredient administered to the patient.

In some embodiments of this method, fenfluramine is used as an adjunctive therapy in a patient. In some embodiments of this method, fenfluramine is used as an adjunctive therapy in a patient with epilepsy or epileptic encephalopathy. In some embodiments of this method, fenfluramine is used as an adjunctive therapy in a patient with Dravet syndrome or Lennox-Gastault syndrome (LGS).

Yet another aspect of the invention contemplates co-administration of an effective dose of one or more co-therapeutic agents with the fenfluramine wherein the co-therapeutic agents can be selected from the group consisting of Brivaracetam, bromides (e.g., Potassium Bromide, Sodium Bromide), Cannabidiol, Carbamazepine, Clonidine, Ergenyl Chrono, Ethosuximide, Felbamate, Fosphenytoin, Lacosamide, Lamotrigine, Levetiracetam, Levocarnitine, Mesuximide, Nitrazepam, Oxcarbazepine, Perampanel, Phenobarbital, Pregabalin, Progabide, Pyridoxine, Rufinamide, Stiripentol, Sultiame, Tizanidine, Topiramate, Valproate semisodium, Valproate sodium, Valproic acid, Verapamil, Zonisamide, and benzodiazepines such as Clobazam, Clonazepam, Diazepam, Ethyl Loflazepate, Lorazepam and Midazolam. Use of a pharmaceutically acceptable salt or base of a co-therapeutic agent is also contemplated.

In another aspect, the subject/patient may have been previously treated with a medication, prior to treatment with fenfluramine, wherein the agent is selected from Acetazolamide, Brivaracetam, Carbamazepine, Clobazam, Clonazepam, Diazepam, Ergenyl Chrono, Ethosuximide, Felbamate, Gabapentin, Lacosamide, Lamotrigine, Levetiracetam, Lorazepam, Mesuximide, Oxcarbazepine, Perampanel, Phenobarbital, Phenytoin, Phenytoin sodium, Pregabalin, Rufinamide, Stiripentol, Sultiame, Topiramate, Valproate semisodium, Valproate sodium, Valproic acid, Vigabatrin, Zonisamide, and a pharmaceutically acceptable salt or base of any of these.

Herein described is the discovery that fenfluramine can be used in the treatment of diseases or disorders affecting cognitive function. Thus, in some aspects, the present disclosure contemplates co-administration of an effective dose of one or more co-therapeutic agent(s) with the fenfluramine.

In some embodiments, the initiating dose of fenfluramine provided is about 0.2 mg/kg/day for between 4 and 7 days with subsequent increases in dosage occurring in increments of about 0.2 mg/kg/day every 4 to 7 days up to a maximum dose of about 0.8 mg/kg/day or to a recommended maximum dose of 30 mg/day. In another aspect a patient is already receiving medication for treating or preventing the seizures which may interact with fenfluramine and initiation of fenfluramine therapy is provided at about 0.2 mg/kg/day for between 4 and 7 days with subsequent increases in dosage occurring in increments of about 0.2 mg/kg/day every 4 to 7 days up to a maximum dose of about 0.5 mg/kg/day or to a recommended maximum dose of 20 mg/day.

In another aspect of the invention, the method may be carried out wherein the effective dose is administered in a form selected from the group consisting of oral, injectable, transdermal, buccal, inhaled, nasal, buccal, rectal, vaginal, or parental, and wherein the formulation is oral, the formulation may be liquid which may be a solution or a suspension may be present within a container closed with a cap connected to a syringe graduated to determine the volume extracted from the container wherein the volume extracted relates to the amount of fenfluramine in a given liquid volume of formulation e.g. one milliliter of formulation contains 2.5 mg of fenfluramine. In another aspect of the invention, the method is administered in a solid oral formulation in the form of a tablet, capsule, lozenge, or sachet.

The method may be carried out as a co treatment with a different pharmaceutically active compound. The method may be carried out in a process wherein the patient is first then subjected to a series of tests to confirm diagnoses of epilepsy.

These and other objects, advantages, and features of the invention will become apparent to those persons skilled in the art upon reading the details of the methods of treating symptoms of epilepsy as more fully described below. The technical information below set out below may in some respects go beyond the scope of the present invention, which is defined by the appended claims. The additional technical information is provided to place the present invention in a broader technical context and to illustrate possible related technical developments.

### BRIEF DESCRIPTION OF THE DRAWINGS

FIG. 1 provides a table of Clinical Global Impression-I (CGI-I) scale values given by an investigator for placebo and treatment groups.
FIG. 2 provides a bar graph showing a visual representation of CGI-I ratings given by an investigator for placebo and treatment groups.
FIG. 3 provides a table of CGI-I values given by a parent or caregiver for placebo and treatment groups.
FIG. 4 provides a bar graph showing a visual representation of CGI-I ratings given by a parent or caregiver for placebo and treatment groups.
FIG. 5 provides a bar graph of CGI-I ratings by the study investigator of patients in a randomized controlled-trial of ZX008 comparing improvement against baseline assessment prior to treatment.
FIG. 6 provides a bar graph of CGI-I ratings by a parent/caregiver of patients in a randomized controlled-trial of ZX008 comparing improvement against baseline assessment prior to treatment.
FIG. 7 provides a bar graph comparing the baseline and final visit CGI-I ratings by the study investigator from patients after 24 months of ZX008 treatment.
FIG. 8 provides a bar graph comparing the baseline and final visit CGI-I ratings by a parent/caregiver from patients after 24 months of ZX008 treatment.

### DETAILED DESCRIPTION OF THE INVENTION

Before the present method, kits and formulations are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described.

It must be noted that as used herein and in the appended claims, the singular forms "a", "an", and "the" include plural referents unless the context clearly dictates otherwise. Thus, for example, reference to "a seizure" includes a plurality of such seizures and reference to "the formulation" includes reference to one or more formulations and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As is the subject of several related patent applications (US 2017-0056344-A1; US 2017-0071949-A1; US 2018-0055789-A1; and US 2018-0092864-A1) and issued patents (9,549,909; 9,610,260; 9,603,814; and 9,603,815), fenfluramine was found to be useful in treating, ameliorating, or minimizing the symptoms of epilepsies, such reducing the number, intensity and/or length of seizures. Fenfluramine is particularly useful in the treatment of epilepsies, and in particular, epileptic encephalopathies such as Dravet syndrome and Lennox-Gastaut syndrome.

Cognitive function may be impaired in patients suffering from symptoms of epilepsy. For example, executive functions (sometimes called "higher level" cognitive functions) can be assessed using lab-based performance measures, or by using the Behavior Rating Inventory of Executive Function (BRIEF).

There are several validated tests used to measure cognitive function, including, but not limited to, the Behavior Rating Inventory of Executive Function (BRIEF), the Wechsler memory scale, the MOCA (Montreal Cognitive Assessment) scale, the Executive Abilities: Measures and Instruments for Neurobehavioral Evaluation and Research (EXAMINER), and other clinical and/or index scales.

As is described hereinbelow, it has been discovered that administration of fenfluramine can be beneficial in treating diseases and disorders affecting cognitive function. In some embodiments, subjects/patients may be administered fenfluramine to improve and treat cognitive function diseases or disorders. In some embodiments, the patient is also being treated for the symptoms of epilepsy. Methods are provided herein for improving and measuring improvement in cognitive function as assessed by scales such as (but not limited to) a Behavior Rating Inventory of Executive Function (BRIEF) score in a patient by administering the BRIEF test to a patient and obtaining a pre-treatment (*i.e*., baseline) BRIEF score, then treating the patient with fenfluramine or a pharmaceutically acceptable salt thereof, and then, sometime after treatment, re-administering the BRIEF test to the patient to obtain a post-treatment score and for determining whether there has been a significant improvement in the BRIEF score.

### Behavior Rating Inventory of Executive Function (BRIEF)

The Behavior Rating Inventory of Executive Function (BRIEF) test was originally developed by Gerard Gioia, Ph.D., Peter Isquith, Ph.D., Steven Guy, Ph.D., and Lauren Kenworthy, Ph.D. (Gioia, et al., (2000). Child Neuropsychology. 6(3):235-238). BRIEF questionnaires are standardized, validated rating scales to measure executive function in children ages 2-18 within the home and school environments. They are designed to provide a standardized method of asking multiple raters about executive functions in daily life in a manner that is not specific to any particular disorder, applicable to a broad range of children.

With regard to the BRIEF, the term executive function is used as an umbrella construct that includes a collection of interrelated cognitive functions that are responsible for purposeful, goal-directed, problem-solving behavior. Specific subdomains that make up this collection of regulatory or management functions include the ability to initiate behavior, inhibit competing actions or stimuli, select relevant task goals, plan and organize a means to solve complex problems, shift problem-solving strategies flexibly when necessary, and monitor and evaluate behavior.

BRIEF-P is for preschool children aged 2-5; BRIEF is for 6 to 18 year olds; BRIEF-SR is specifically for self-reports of adolescents aged 11-18; and BRIEF-A is for self/informant-reports of adults aged 18-90.

Because it is not disorder-specific, the BRIEF may be used to assess executive function behaviors in children and adolescents experiencing a wide range of difficulties, such as those related to learning disabilities and attentional disorders, traumatic brain injuries, lead exposure, pervasive developmental disorders, depression, and other developmental, neurological, psychiatric, and medical conditions.

### Test format

In the BRIEF test, the parent/caregiver and/or the patient (*e.g.,* child or adolescent self-reporting) responds to a series of statements or questions in the form of questionnaires, where an answer of "N" = Never a problem, "S"= Sometimes a problem, and "O"= Often a problem are used to indicate that the behavior being described or assessed has occurred in the past 6 months.

Each questionnaire of the BRIEF parent- and teacher- rating form contains 86 items in eight non-overlapping clinical scales and two validity scales. Based on theoretical and empirical analyses (reviewed in chapter 5 of Gioia, *Ibid*.)*,* the clinical scales combine to form two indexes: a) Behavioral Regulation (three scales) and b) Metacognition (five scales), as well as a Global Executive Composite (GEC), which is a summary score that takes into account all eight clinical scales of the BRIEF and represents the child's overall executive function. There are also two validity scales to measure Negativity and Inconsistency of responses. Scores on the Negativity scale measures the extent to which the respondent answered selected items in an unusually negative manner whereas scores on the Inconsistency scale indicate the extent to which the respondent answered similar items in an inconsistent manner.

The Behavioral Regulation Index (BRI) represents a child's ability to shift cognitive set and modulate emotions and behavior via appropriate inhibitory control. It is comprised of the Inhibit, Shift, and Emotional Control scales. Appropriate behavioral regulation is likely to be a precursor to appropriate metacognitive problem solving. Behavioral regulation enables the metacognitive processes to successfully guide active, systematic problem solving, and more generally, supports appropriate self-regulation.

The Metacognition Index (MI) represents the child's ability to initiate, plan, organize, and sustain future-oriented problem solving in working memory. This index is interpreted as the ability to cognitively self-manage tasks and reflects the child's ability to monitor his or her performance. The MI relates directly to a child's ability to actively problem solve in a variety of contexts. It is comprised of the Initiate, Working Memory, Plan/Organize, Organization of Materials, and Monitor scales.

### Behavioral regulation scales

- *Inhibit:* measures ability to control impulses (inhibitory control) and to stop engaging in a behavior.
- *Shift:* measures ability to move freely from one activity / situation to another; transition; to tolerate change; to switch or alternate attention; problem-solving flexibility.
- *Emotional Control:* measures ability to modulate emotional responses appropriately.

### Metacognition scales

- *Initiate:* measures ability to begin an activity and to independently generate ideas or problem-solving strategies.
- *Working Memory:* measures ability to hold information in mind for purpose of completing a task, when encoding information, or when generating goals/plans in a sequential manner.
- *Plan*/*Organize*: measures ability to anticipate future events; to set goals; to develop steps; to grasp main ideas; to organize and understand the main points in written or verbal presentations.
- *Organization of Materials:* measures ability to put order in work, play and storage spaces (e.g., desks, lockers, backpacks, and bedrooms).
- *Monitor:* measures ability to check work and to assess one's own performance; ability to keep track of the effect of one's own behavior on other people.

### Administration

The BRIEF is very simple to administer and only requires a copy of the form and a pencil. The parent form is filled out by a parent (preferably by both parents). One preferred criterion is they should have had recent contact with the child over the past six months. Similarly, the teacher form can be filled out by any adult (teacher or aide) who has had extended contact with the child in a school setting during the past month. Multiple ratings across classrooms are strongly recommended, as they are useful for comparison purposes.

### Reliability and validity

Questions selected for inclusion in the BRIEF were determined based on inter-rater reliability correlations and item-total correlations that had the highest probability of being informative for the clinician. The BRIEF has demonstrated good reliability, with high test-retest reliability (rs ≈ .88 for teachers, .82 for parents) internal consistency (Cronbach's alphas ≈ .80 - .98), and moderate correlations between parent and teacher ratings (rs ≈ .32 - .34). Evidence for the convergent and divergent aspects of the BRIEF's validity comes through its correlation with other measures of emotional and behavioral functioning. The BRIEF has also demonstrated utility in differentiating clinical and non-clinical children and adolescents with attention deficit/hyperactivity disorder (ADHD).

### Scoring and interpretation

Raw scores for all scales of the BRIEF questionnaire can be computed with the Software Portfolio (BRIEF-SP), which provides separate normative tables for both the Parent and Teacher Forms, figure T scores, percentiles, and 90% confidence intervals for four developmental age groups (5-18 years) by gender of the child. T scores provide information about the child's individual scores relative to the scores of other respondents in the standardization sample. Percentiles represent the percentage of children in the standardization sample who fall below a given raw score.

Clinical information gathered from the BRIEF questionnaire is best understood within the context of a full assessment that includes a description of the history of the child and the family and observations of the child's behavior. Accordingly, high scores obtained on the BRIEF suggest a higher level of dysfunction in a specific domain of executive functions. Particular attention should also be paid to the Inconsistency scale given that score equal or higher than 7 is indicative of a high degree of inconsistency in rater response.

### Uses

The BRIEF is useful for evaluating children with a variety of disorders and disabilities. Specifically, it is often used for assessing executive functioning in children with developmental and/or acquired neurological conditions including: learning disabilities, Tourette syndrome, traumatic brain injury, pervasive developmental disorders, high functioning autism, low birth weight. The BRIEF is most often used to assess Attention Deficit/Hyperactivity Disorder.

### Attention deficit/hyperactivity disorder

The BRIEF is often used to evaluate ADHD in children and has been shown to be superior to other rating systems such as the Behavior Assessment System for Children (BASC) as it taps into unique behaviors typically associated with the disorder (e.g., working memory, metacognitive skills).

McCandless & O'Laughlin (2007) found that the Metacognitive and Behavioral Regulation scales of the BRIEF are clinically useful for identifying children with and without ADHD. Specifically, the Metacognitive Scale (Working Memory subscale) is useful for identifying the presence of ADHD whereas the Behavioral Regulation scale (Inhibit subscale) has demonstrated clinical utility at distinguishing between the inattentive and combined (i.e., inattentive and hyperactive) subtypes of the disorder (McCandless & O' Laughlin (2007) Journal of Attention Disorders. 10(4):381-389).

The BRIEF has also been useful for highlighting differences between ADHD and other diagnoses. For example, parent reports on the BRIEF for children (ages 6-11) who had a diagnosis of ADHD, ADHD and reading disorder (RD), RD only, or no diagnosis. Children with ADHD demonstrated higher scores on all of the BRIEF scales compared to children with no formal diagnosis were examined; children with a reading disorder showed greater difficulties on the Working Memory and the Plan/Organize subscales of the Metacognitive Scale.

According to an aspect of the present disclosure, fenfluramine treatment continues over a period of time and in amounts effective to improve the cognitive function, which can be assessed via improvements on one of several validated scales, such as (but not limited to) the Behavior Rating Inventory of Executive Function (BRIEF), the Wechsler memory scale, the MOCA (Montreal Cognitive Assessment) scale, the Executive Abilities: Measures and Instruments for Neurobehavioral Evaluation and Research (EXAMINER), or other validated clinical and/or index scales which measure cognitive function.

When the BRIEF test is administered to the parent/caregiver and/or the patient (e.g., child or adolescent self-reporting), the responses indicate whether certain cognitive functions measured within the two main indices (Behavioral Regulation Index (BRI) and Metacognition Index (MI)) have occurred in the past 6 months. The BRI includes three scales (Inhibit, Shift, and Emotional Control) and the MI includes five scales (Initiate, Working Memory, Plan/Organize, Organization of Materials, and Monitor scales).

The rating of Clinical Global Impression, CGI, was developed for use in NIMH-sponsored clinical trials to provide a brief, stand-alone assessment of the clinician's view of the patient's global functioning prior to and after initiating a study medication. The CGI provides an overall clinician-determined summary measure that takes into account all available information, including a knowledge of the patient's history, psychosocial circumstances, symptoms, behavior, and the impact of the symptoms on the patient's ability to function.

The CGI actually comprises two companion one-item measures evaluating the following: (a) severity of psychopathology from 1 to 7 and (b) change from the initiation of treatment on a similar seven-point scale. Subsequent to a clinical evaluation, the CGI form can be completed in less than a minute by an experienced rater. In practice, the CGI captures clinical impressions which encompass more than symptom checklists. It is readily understandable and can be used with relative ease by the non-researcher clinician. Beyond that, the CGI can track clinical progress across time and has been shown to correlate with longer, more time-consuming rating instruments across a wide range of psychiatric diagnoses.

In clinical practice, the CGI is administered by an experienced clinician who is familiar with the disease under study and the likely progression of treatment. Consequently, the CGI rater can make an expert clinical global judgment about the severity of the illness across various time points within the context of that clinical experience. The clinician makes a judgment about the total picture of the patient at each visit: the illness severity, the patient's level of distress and other aspects of impairment, and the impact of the illness on functioning. The CGI is rated without regard to the clinician's belief that any clinical changes are or are not due to medication and without consideration of the etiology of the symptoms.

The CGI-I rating may also be made by a parent or caregiver or anyone who observes and interacts with the patient on a frequent basis. Such ratings are not based on experience with other patients having the disease but are made on observed changes and fluctuations in the individual patient's mental and psychiatric function observed more frequently than can be made by the clinical investigator or treating doctor.

In patients treated with fenfluramine, a notable and unexpected improvement in score on cognition tests was observed. For example, upon taking the BRIEF test, patients taking fenfluramine had improved scores on several scales within the BRI and/or MI scales, with a trend noted that indicated improvement in cognitive function, which was in opposition to the trend toward reduced cognitive function observed on these scales in placebo-treated epileptic patients.

In patients treated with fenfluramine, a notable and unexpected improvement in scores of the Clinical Global Impression, which are statistically relevant were demonstrated in patients during clinical studies described herein, including increases in ratings of much improved and very much improved continuing over months and years of treatment. The improvement in CGI as a treatment effect is demonstrated via percentages of patients exhibiting improvement.

As described herein, the phrase "improvement in cognitive function" means that, after fenfluramine treatment, a patient's score(s) on a validated measure of cognitive function, such as the BRIEF, the Wechsler memory scale, the MOCA, and/or the EXAMINER scales, improves as compared to baseline score(s). In some embodiments, the improvement in cognitive function is measured as percent improvement, and is statistically significant. In some embodiments, at least one score is improved by 5% or more, 10% or more, 15% or more, 25% or more, 50% or more, or 75% or more.

As described herein, the phrase "improvement in clinical global impression" means that, after fenfluramine treatment, a patient's score(s) on a validated measure of nervous system function, such as and CGI-I improves as compared to the CGI-S baseline score(s). In some embodiments, the improvement in function is measured by assigning a rating score at a baseline and re-evaluating the patient for a new rating score. Improvement is indicated by achieving an improved ranking of at least one level above the previous ranking. Nervous system function improvements may include assessments of symptoms relating to behavior, cognition, motor abilities (ataxias, tremors, gait abnormalities), speech, alertness or frequency or severity of seizures, depending on the patient or disease state receiving treatment.

To avoid doubt, the term "prevention" of seizures means the total or partial prevention (inhibition) of seizures. Ideally, the methods of the present invention result in a total prevention of seizures. However, the invention also encompasses methods in which the instances of seizures are decreased in frequency by at least 40%, at least 50%, at least 55%, at least 60%, at least 65%, at least 70%, at least 75%, at least 80%, at least 85%, or at least 90%. In addition, the invention also encompasses methods in which the instances of seizures are decreased in duration or severity by at least 40%, at least 50%, at least 55%, at least 60%, at least 65%, at least 70%, at least 75%, at least 80%, at least 85%, or at least 90%.

Serotonin (5-HT) is a monoaminergic neurotransmitter that is believed to modulate numerous sensory, motor and behavioral processes in the mammalian nervous system. These diverse responses are elicited through the activation of a large family of receptor subtypes. The complexity of this signaling system and the paucity of selective drugs have made it difficult to define specific roles for 5-HT receptor subtypes, or to determine how serotonergic drugs modulate mood and behavior. Of the many subtypes of serotonin receptors, the 1B and 2C subtypes are most strongly implicated in modulating feeding and body weight, and these receptors are expressed in hypothalamic regions believed to be involved in food intake regulation. Both 1B and 2C receptor agonists have been found to suppress feeding in rodents, and 2C receptor knockout mice display chronic hyperphagia and obesity. Furthermore, knockout mice lacking functional 5-HT₂C receptors (previously termed 5-HT₁C) were found to be hyperphagic, which led to obesity, partial leptin resistance, increased adipose deposition, insulin resistance, and impaired glucose tolerance. Thus, the 5-HT₂C receptor is reportedly involved in the serotonergic control of food intake and body weight. The knockout mice were also prone to spontaneous death from seizures, suggesting that 5-HT₂C receptors also mediate tonic inhibition of neuronal network excitability. (Tecott LH, et al. Eating disorder and epilepsy in mice lacking 5-HT2C serotonin receptors. Nature. 1995, 374(6522):542-6).

Fenfluramine, i.e. 3-trifluoromethyl-N-ethylamphetamine is an amphetamine derivative having the structure:
Structure 1

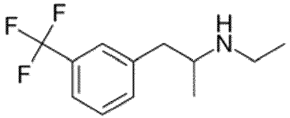

### Systematic (IUPAC) name (RS)-N-ethyl- 1-[3-(trifluoromethyl)phenyl]propan- 2-amine

Fenfluramine is a racemic mixture of two enantiomers, dexfenfluramine and levofenfluramine, and has been reported to increase the circulating levels of serotonin, a neurotransmitter that regulates mood, appetite and other functions.

Fenfluramine was first marketed in the US in 1973 to treat obesity. However, in 1997, it was withdrawn from the US and global market as its use was associated with the onset of cardiac valvulopathy and pulmonary hypertension. Subsequently, the drug was withdrawn from sale globally and is no longer indicated for use in any therapeutic area. Without being bound by theory, the adverse effects associated with the use of fenfluramine as an anorexic agent are thought to be attributable to the interaction of fenfluramine's major metabolite norfenfluramine with the 5-HT₂B receptor, which is associated with heart valvulopathy.

Fenfluramine and its major metabolite, norfenfluramine, were reported to be potent substrates for norepinephrine transporters. (Rothman, et al., J. Pharmacol. Exp. Ther. 305(3):1191-9). Fenfluramine also acts as a norepinephrine releasing agent to a lesser extent, particularly via its active metabolite norfenfluramine. Fenfluramine causes the release of serotonin by disrupting vesicular storage of the neurotransmitter, and reversing serotonin transporter function. At high concentrations, norfenfluramine also acts as a dopamine releasing agent, and so fenfluramine may do this at very high doses as well. In addition to monoamine release, while fenfluramine binds only very weakly to the serotonin 5-HT₂ receptors, norfenfluramine binds to and activates the serotonin 5-HT₂B and 5-HT₂C receptors with high affinity and the serotonin 5-HT₂A receptor with moderate affinity. The result of the increased serotonergic and noradrenergic neurotransmission is a feeling of fullness and reduced appetite.

Despite past cardiovascular safety concerns that arose when high doses of fenfluramine were used for treatment of adult obesity, attempts have been made to identify further therapeutic uses for that product, while weighing the known cardiovascular risks of fenfluramine against potential therapeutic benefits. One disorder for which new treatment options are sorely needed is epilepsy, and in particular, epilepsy syndromes which are refractory to known treatments. Epilepsy is a functional disturbance of the central nervous system (CNS) induced by abnormal electrical discharges and marked by a susceptibility to recurrent seizures. There are numerous causes of epilepsy including, but not limited to birth trauma, perinatal infection, anoxia, infectious diseases, ingestion of toxins, tumors of the brain, inherited disorders or degenerative disease, head injury or trauma, metabolic disorders, cerebrovascular accident and alcohol withdrawal.

Although some antiepileptic drugs have been developed, approximately one third of patients with epilepsy are refractory to treatment. Therefore, the search for new mechanisms and medications that can regulate cellular excitability continues. Three drugs that are especially effective for partial onset seizures are vigabatrin, a selective and irreversible GABA-transaminase inhibitor that greatly increases whole-brain levels of GABA; tiagabine, a potent inhibitor of GABA uptake into neurons and glial cells; and topiramate, which is believed to produce its antiepileptic effect through several mechanisms, including modification of Na⁺- dependent and/or Ca²⁺-dependent action potentials, enhancement of GABA-mediated Cl⁻ fluxes into neurons, and inhibition of kainate-mediated conductance at glutamate receptors of the AMPA/kainate type. (Ängehagen, et al., 2003, Neurochemical Research, 28(2):333-340).

Historically, investigation of fenfluramine's efficacy in epilepsy patients led to a common paradigm, i.e., that fenfluramine's primary effects were on behaviors that caused or induced seizures, not treating or preventing the seizure itself.

For example, Aicardi and Gastaut (New England Journal of Medicine (1985), 313:1419 and Archives of Neurology (1988) 45:923-925) reported four cases of self-induced photosensitive seizures, *i.e.,* seizures caused by patients purposely staring into bright lights or the sun, which were found to respond to treatment with fenfluramine.

Clemens, in Epilepsy Research (1988) 2:340-343, reported a case study wherein a boy suffering pattern sensitivity-induced seizures that were resistant to anticonvulsive treatment was treated with fenfluramine to curb the patient's compulsive seizure-inducing behavior. Fenfluramine reportedly successfully terminated these self-induced seizures. Clemens concluded that this was because fenfluramine blocked the photosensitive triggering mechanism, and, secondarily, by diminishing the pathological drive toward the seizure triggering behavior/compulsion, *i.e.,* not by treating the seizure itself.

In Neuropaediatrics, (1996); 27(4):171-173, Boel and Casaer reported on a study on the effects of fenfluramine on children with refractory epilepsy, all of whom exhibited compulsive seizure-inducing behavior. They observed that when fenfluramine was administered at a dose of 0.5 to 1 mg/kg/day, this resulted in a reduction in the number of seizures experienced by the patients, and concluded that "this drug could have significant anti-epileptic activity in a selected group of young patients with idiopathy or symptomatic generalized epilepsy, namely, children with self-induced seizures." The authors noted that "[i]t may well be that fenfluramine has no direct antiepileptic activity but acts through its effect on the compulsion to induce seizures." Hence, the authors suggested that fenfluramine affected behavior and not the seizure itself.

In a letter to *Epilepsia,* published in that journal (Epilepsia, 43(2):205-206, 2002), Boel and Casaer commented that fenfluramine appeared to be of therapeutic benefit in patients with intractable epilepsy and self-induced seizures. However, the authors did not attribute fenfluramine's efficacy to generalized anti-seizure activity.

A large number of subtypes of epilepsy have been characterized, each with its own unique clinical symptoms, signs, and phenotype, underlying pathophysiology and distinct responses to different treatments. The present disclosure has applicability with respect to a range of different types of epilepsies and epilepsy subtypes, including Dravet syndrome, Doose syndrome, infantile spasms, and Lennox-Gastaut syndrome. There are a large number of subtypes of epilepsy that have been characterized. For example, the most recent classification system, and one that is widely accepted in the art, is that adopted by the International League Against Epilepsy's ("ILAE") Commission on Classification and Terminology [*See e.g.,,* Berg et al., "Revised terminology and concepts for organization of seizures," Epilepsia, 51(4):676-685 (2010*)*]:
I. Electroclinical syndromes arranged by age at onset:
   A. Neonatal period (1. Benign familial neonatal epilepsy (BFNE), 2. Early myoclonic encephalopathy (EME), 3. Ohtahara syndrome),
   B. Infancy (1. Epilepsy of infancy with migrating focal seizures, 2. West syndrome, 3. Myoclonic epilepsy in infancy (MEI), 4. Benign infantile epilepsy, 5. Benign familial infantile epilepsy, 6. Dravet syndrome, 7. Myoclonic encephalopathy in nonprogressive disorders),
   C. Childhood (1. Febrile seizures plus (FS+) (can start in infancy), 2. Panayiotopoulos syndrome, 3. Epilepsy with myoclonic atonic (previously astatic) seizures, 4. Benign epilepsy with centrotemporal spikes (BECTS), 5. Autosomal- dominant nocturnal frontal lobe epilepsy (ADNFLE), 6. Late onset childhood occipital epilepsy (Gastaut type), 7. Epilepsy with myoclonic absences, 8. Lennox-Gastaut syndrome, 9. Epileptic encephalopathy with continuous spike-and-wave during sleep (CSWS), 10. Landau-Kleffner syndrome (LKS), 11. Childhood absence epilepsy (CAE));
   D. Adolescence - Adult (1. Juvenile absence epilepsy (JAE), 2. Juvenile myoclonic epilepsy (JME), 3. Epilepsy with generalized tonic-clonic seizures alone, 4. Progressive myoclonus epilepsies (PME), 5. Autosomal dominant epilepsy with auditory features (ADEAF), 6. Other familial temporal lobe epilepsies,
   E. Less specific age relationship (1. Familial focal epilepsy with variable foci (childhood to adult), 2. Reflex epilepsies);
II. Distinctive constellations:
   A. Mesial temporal lobe epilepsy with hippocampal sclerosis (MTLE with HS),
   B. Rasmussen syndrome,
   C. Gelastic seizures with hypothalamic hamartoma,
   D. Hemiconvulsion-hemiplegia-epilepsy,
   E. Other epilepsies, distinguished by 1. presumed cause (presence or absence of a known structural or metabolic condition, then 2. primary mode of seizure onset (generalized vs. focal);
III. Epilepsies attributed to and organized by structural-metabolic causes:
   A. Malformations of cortical development (hemimegalencephaly, heterotopias, etc.),
   B. Neurocutaneous syndromes (tuberous sclerosis complex, Sturge-Weber, etc.),
   C. Tumor,
   D. Infection,
   E. Trauma;
IV. Angioma: A. Perinatal insults, B. Stroke, C. Other causes;
V. Epilepsies of unknown cause;
VI. Conditions with epileptic seizures that are traditionally not diagnosed as a form of epilepsy per se; A. Benign neonatal seizures (BNS); and B. Febrile seizures (FS).

See Berg et. al, "Revised terminology and concepts for organization of seizures," Epilepsia, 51(4):676-685 (2010)).

Part V of the ILAE classification scheme underscores the fact that the list is far from complete, and that there are still subtypes of epilepsy that have not yet been fully characterized, or that remain unrecognized as distinct syndromes.

Those skilled in the art will recognize that different subtypes of epilepsy are triggered by different stimuli, are controlled by different biological pathways, and have different causes, whether genetic, environmental, and/or due to disease or injury of the brain. In other words, the skilled artisan will recognize that teachings relating to one epileptic subtype are most commonly not necessarily applicable to other subtypes. Of particular importance is the fact that there are a large number of compounds that are used to treat different types of epilepsy, and different epilepsy subtypes respond differently to different anticonvulsant drugs. That is, while a particular drug may be effective against one form of epilepsy, it may be wholly ineffective against others, or even contra-indicated due to exacerbation of symptoms, such as worsening the frequency and severity of the seizures. As a result, efficacy of a particular drug with respect to a particular type of epilepsy is wholly unpredictable, and the discovery that a particular drug is effective in treating in treating a type of epilepsy for which that drug was not previously known to be effective is nearly always surprising, even in cases where the drug is known to be effective against another epilepsy type. Furthermore, as will be described in detail below, effective treatment of a form of epilepsy with fenfluramine can contra-indicate co-administration of and/or treatment with certain other therapeutic agents.

One form of epilepsy that may be treated with fenfluramine is known as Dravet syndrome. Dravet syndrome is a rare and catastrophic form of intractable epilepsy that begins in infancy. Children with Dravet syndrome do not outgrow the condition, and it affects every aspect of their daily lives, according to Dravet Foundation.org. Children with the seizure disorder also face behavior and developmental delays; movement and balance issues; bone problems; delayed language and speech problems; growth and nutrition issues; trouble sleeping; chronic infections; and autonomic dysfunctions, such as problems regulating body temperature, however the occurrence of these symptoms is not uniform and some patients may be affected with some symptoms and not others and are affected to varying degrees. People with this disorder also have a higher risk of death during seizures.

Initially, in the first year of life the patient with Dravet syndrome experiences prolonged seizures. In their second year, additional types of seizure begin to occur and this typically coincides with a developmental decline, possibly due to repeated seizures causing brain damage such as cerebral hypoxia.

Cognitive deficits and behavioral disturbances are a common trait in Dravet patients. The deficits are relatively homogeneous in quality but of different degrees. Developmental delay becomes progressively evident from the second year on. Generally, children start walking at a normal age, but an unsteady gait develops over an unusually long period. Language also starts at a normal age, but progresses very slowly, and many patients do not reach the stage of constructing elementary sentences. Patients' fine motor abilities do not develop well. They are disturbed by segmental myoclonus and by poor eye-hand coordination. Sleep disturbances are often present. Even patients with milder cognitive impairment may not be able to draw a design and may write only by printing letters. Lack of attention is one of the major factors responsible for the learning disabilities, as well as for hyperactivity and recalcitrant behavior. Affected children may be restless, not listen to adults, and not show interest in playing with educational toys or participating in the usual activities of their age group. Conversely, affected children are often able to complete puzzles and to watch cartoons repetitively. Not all these traits are present in all patients and the traits tend to be less severe in those with a recent diagnosis (Dravet, C. Epilepsia, 52(Suppl. 2):3-9, 2011). In the months following seizure onset, parents' observations and neuropsychological testing confirm developmental slowing and, in some cases, decline of cognitive measures and/or significant fluctuations of cognitive measures are present. (Ragona, F., Epilepsia, 52(Suppl. 2):39-43, 2011).

Children with Dravet syndrome are likely to experience multiple seizures per day. Epileptic seizures are far more likely to result in death in sufferers of Dravet syndrome; approximately 10 to 15% of patients diagnosed with Dravet syndrome die in childhood, in some cases between two and four years of age. The mean age at death of patients is reported to be 8.7 ± 9.8 years (SD), with 73% of deaths occurring before the age of 10 years, and 93% before the age of 20. Additionally, patients are at risk of numerous associated conditions including orthopedic developmental issues, impaired growth and chronic infections.

Of particular concern, children with Dravet syndrome are susceptible to episodes of Status Epilepticus, a convulsive seizure lasting longer than 5 minutes. This severe and intractable condition is categorized as a medical emergency requiring immediate medical intervention, typically involving hospitalization for intravenous anticonvulsant medication and/or medically-induced coma. Status epilepticus can be fatal. It can also be associated with severe cerebral hypoxia, possibly leading to damage to brain tissue. Frequent hospitalizations of children with Dravet syndrome are clearly distressing, not only to the patient but also to family and caregivers.

The cost of care for Dravet syndrome patients is also high as the affected children require constant supervision and many require institutionalization as they reach teenage years.

Seizures in Dravet syndrome can be difficult to manage but may be reduced by anticonvulsant medications such as clobazam, stiripentol, topiramate and valproate. Because the course of the disorder varies from individual to individual, treatment protocols may vary. A diet high in fats and low in carbohydrates may also be beneficial, known as a ketogenic diet. Although diet adjustment can help, it does not eliminate the symptoms. Until a better form of treatment or cure is discovered, those with this disease will have myoclonic epilepsy for the rest of their lives.

At present, although a number of anticonvulsant therapies can be employed to reduce the instance of seizures in patients with Dravet syndrome, the results obtained with such therapies are typically poor and those therapies only effect partial cessation of seizures at best. Seizures associated with Dravet syndrome are typically resistant to conventional treatments. Further, many anticonvulsants such as clobazam and clonazepam have undesirable side effects, which are particularly acute and prominent in pediatric patients.

Non-epileptic brains have a natural balance of excitation (that can evoke seizures) and inhibition (that can reduce seizures). Sodium channel blockers preferentially affect sodium channels at a specific stage of their cycle of rest, activation and inactivation, often by delaying the recovery from the inactivated state, thereby producing a cumulative reduction of Na+. Sodium channel blockers are widely used in treating epilepsies that are caused by too much excitatory neurotransmission (with the exception of SCN1A-mutation-related epilepsies). In some epilepsies, sodium channel blockers may work to correct an imbalance of excitatory and/or inhibitory neurotransmitter(s) to make seizures less likely to occur. However, while sodium channel blockers are beneficial in treatment of some epilepsies this class of drugs are contra-indicated in Dravet syndrome, as sodium channel blockers have been found to lead to a greater incidence of seizures in almost all Dravet syndrome patients.

Without being bound by theory, approximately 70-90% of patients with Dravet syndrome have nonsense mutations in the SCN1A gene which encodes the alpha-1 subunit of the sodium ion channel (Nav1.1), containing 2,009 amino acids, and is primarily expressed in inhibitory neurons. At least 70 - 80% of patients with Dravet syndrome have SCN1A mutations in the gene's exon which cause a loss of sodium channel function. Dravet has suggested as high as 85% have an SNC1A mutation (Dravet C., The core Dravet syndrome phenotype. Epilepsia 2011; 52 (Suppl 2): 3-9). Some researchers predict that since only coding regions of the SCN1A gene are sequenced it is likely that many of the remaining patients harbor mutations in regulatory regions of the gene (outside of the coding sequences) that impair or prevent expression of this channel. Mice with loss-of function mutations in NaV1.1 channels have severely impaired sodium currents and action potential firing in hippocampal GABAergic inhibitory neurons without detectable effect on the excitatory pyramidal neurons, which would cause hyperexcitability and contribute to the seizures observed in Dravet Syndrome. Complete loss-of-function mutations in NaV1, encoded by SCNA1, cause Dravet Syndrome, which involves severe, intractable epilepsy and comorbidities of ataxia, gait abnormalities, problems with language and speech, sleep disturbances, and cognitive impairment.

In the impaired Nav1.1 channels, sodium currents and action potential firing are similarly impaired in the GABAergic Purkinje neurons in the cerebellum, which likely contributes to ataxia, and in the reticular nucleus of the thalamus and the suprachiasmatic nucleus of the hypothalamus, which likely contribute to circadian rhythm disturbances and sleep disorder. (Noebels et. al., Jasper's Basic Mechanisms of the Epilepsies, 4th edition, Bethesda (Md.): National Center for Biotechnology Information (US); 2012).

Since mild loss-of-function mutations in NaV1.1 channels present a milder epilepsy phenotype called Familial Febrile Seizures, a unified loss-of-function hypothesis has been proposed for the spectrum of epilepsy syndromes caused by genetic changes in NaV1.1 channels: mild impairment predisposes to febrile seizures, intermediate impairment leads to GEFS+ epilepsy, and severe loss of function causes the intractable seizures and co-morbidities of Dravet Syndrome. (Catterall WA, et al., NaV1.1 channels and epilepsy. J. Physiol. 2010; 588: 1849-59).

Experts in the field were surprised that haploinsufficiency (in which only one functional copy of the gene, as opposed to the usual two) is not enough to maintain healthy neuronal network function of a NaV channel causes epilepsy, because reduced sodium current should lead to hypoexcitability rather than hyperexcitability. The mechanistic basis for hyperexcitability and co-morbidities in Dravet Syndrome was studied using an animal model generated by targeted deletion or mutation of the SCN1A gene in mouse. Homozygous null NaV1.1(-/-) mice developed ataxia and died on postnatal day (P)15 (Ogiwara, et al., 2007, J. Neurosci. 27:5903-5914., Yu, et al. 2006, Nat. Neurosci. 9:1142-1149). Heterozygous NaV1.1(+/-) mice exhibited spontaneous seizures and sporadic deaths beginning after P21, with a striking dependence on genetic background.

The loss of NaV1.1 did not change voltage-dependent activation or inactivation of sodium channels in hippocampal neurons, however, the sodium current density was substantially reduced in inhibitory interneurons of NaV1.1(+/-) and NaV1.1(-/-) mice, but not in their excitatory pyramidal neurons. This reduction in sodium current caused a loss of sustained high-frequency firing of action potentials in hippocampal and cortical interneurons, thereby impairing their *in vivo* inhibitory function that depends on generation of high-frequency bursts of action potentials.

Given that sodium channel blockers are reported to prevent seizure activity in some epilepsies, treating Dravet patients lacking SCN1A function with sodium channel blockers might be expected to prevent seizures in patients with Dravet syndrome. Instead, treatment of patients with Dravet syndrome with sodium channel blockers leads to increased seizure activity. One explanation may be that, in Dravet syndrome patients, the problem is not too much excitation, but rather too little inhibition. Therefore, giving sodium channel blocking drugs to Dravet syndrome patients decreases the amount of inhibitory neurotransmitters in the brain, tipping the balance toward more seizure activity. Thus, certain anticonvulsant drugs classed as Sodium Channel Blockers are now known to make seizures worse in most Dravet patients. Thus, according to the present disclosure, sodium channel blocker drugs may be contraindicated in connection with the present invention may include the following: phenytoin, carbamazepine, gabapentin, lamotrigine, oxcarbazepine, rufinamide, lacosamide, eslicarbazepine acetate, and fosphenytoin. Similarly, selective GABA reuptake inhibitors / GABA-transaminase inhibitors including tiagabine and vigabatrin should be avoided in Dravet syndrome. A double-blind placebo trial was performed using stiripentol, a GABAergic agent and as a positive allosteric modulator of GABAA receptor. This drug showed efficacy in trials, found to improve focal refractory epilepsy, as well as Dravet's syndrome, supplemented with clobazam and valproate.

Stiripentol was found to reduce tonic-clonic seizure rate by 70%, and is approved in Europe, Canada, Japan and Australia but not in the US, for the treatment of Dravet syndrome. Although stiripentol has some anticonvulsant activity on its own, it acts primarily by inhibiting the metabolism of other anticonvulsants thereby prolonging their activity. It is labeled for use in conjunction with clobazam and valproate. However, concerns remain regarding the use of stiripentol due to its inhibitory effect on hepatic cytochrome P450 enzymes. Further, the interactions of stiripentol with a large number of drugs means that combination therapy (which is typically required for patients with Dravet syndrome) is problematic. Additionally, the effectiveness of stiripentol is limited, with few if any patients ever becoming seizure free.

Polypharmacy, the use of two or more anti-epileptic drugs, for the treatment of Dravet syndrome can result in a significant patient burden, as the side effects, or adverse events from the multiple medications can be additive, and result in limiting the effectiveness of the therapy due to intolerability; in other words the small benefit of a medication may not outweigh the risk or negative effects the drug is having on the patient.

In cases with more drug resistant seizures, treatment with benzodiazepines, valproate, and/or stiripentol, or bromides and topiramate as well as non-pharmaceutical interventions such as a ketogenic diet and vagus nerve stimulation are used as alternative treatments. Treatments also include cognitive rehabilitation through psychomotor and speech therapy. In addition, valproate is often administered to prevent recurrence of febrile seizures and benzodiazapine is used for long lasting seizures, but these treatments are usually insufficient.

Various compounds have been tested for treating different types of epilepsy, and different epilepsy subtypes respond differently to different anticonvulsant drugs. For example, cannabidiol (CBD) has received orphan drug status in the United States, for treatment of Dravet syndrome; cannabidiol has been studied for treatment of drug-resistant seizures in Dravet syndrome and was reported to reduce convulsive-seizure frequency (Devinsky, et al., 2017, NEJM 376(21):2011-2020*).*

As another exemplary form of epilepsy that may be treated with fenfluramine is Lennox-Gastaut syndrome (LGS). LGS was first described in 1960, and named for neurologists William G. Lennox (Boston, USA) and Henri Gastaut (Marseille, France). It is a difficult-to-treat form of childhood-onset epilepsy that most often appears between the second and sixth year of life, although it can occur at an earlier or later age. LGS is characterized by frequent seizures and different seizure types; it is typically accompanied by developmental delay and psychological and behavioral problems. In children, common causes of LGS include perinatal brain injury, brain malformations such as tuberous sclerosis or cortical dysplasia, CNS infection, and degenerative or metabolic disorders of the nervous system.

Daily multiple seizures of different types are typical in LGS. Also typical is the broad range of seizures that can occur. The most common seizure types are tonic-axial, atonic, and absence seizures, but myoclonic, generalized tonic-clonic, and focal seizures can also occur in any LGS patient. Atonic, atypical absence, tonic, focal, and tonic-clonic seizures are also common. Additionally, many LGS patients will have status epilepticus, often of the nonconvulsive type, which is characterized by dizziness, apathy, and unresponsiveness. Further, most patients have atonic seizures, also called drop seizures, which cause their muscles to go limp and result in the patient suddenly and unexpectedly to fall to the ground, often causing significant injury, which is why patients often wear a helmet to prevent head injury.

In addition to daily multiple seizures of various types, children with LGS frequently have arrested/slowed psycho-motor development and behavior disorders.

The syndrome is also characterized by a specific finding on electroencephalogram (EEG), specifically an interictal *(i.e.,* between-seizures) slow spike-wave complexes and fast activity during sleep.

### Diagnosis

LGS is a syndrome and hence its diagnosis is based on the presence of specific clinical symptoms, signs, and laboratory tests. LGS is typically identified by a triad of features including multiple types of seizures, mental retardation or regression and abnormal EEG with generalized slow spike and wave discharges. Physicians use EEG to assist in diagnosing LGS. Diagnosis may be difficult at the onset of the initial symptom(s) because the triad of features associated with LGS, such as tonic seizures, may not be fully established, and EEG during sleep is required to confirm the condition. Thus, even though there may be overlap in clinical presentation with other epilepsies, LGS is agreed to be a well-defined distinct diagnosis by both the International League Against Epilepsy (ILAE), considered the world's leading expert medical society on epilepsy, and the FDA.

The diagnosis of LGS is more obvious when the patient suffers frequent and manifold seizures, with the classic pattern on the electro-encephalogram (EEG), *i.e.,* a slowed rhythm with Spike-wave-pattern, or with a multifocal and generalizing sharp-slow-wave-discharge at 1.5-2.5 Hz. During sleep, tonic patterns (fast activity) can often be seen.

General medical investigation usually reveals developmental delay and cognitive deficiencies in children with LGS. These may precede development of seizures, or require up to two years after the seizures begin, in order to become apparent.

There may be multiple etiologies for LGS, including genetic, structural, metabolic or unknown. Approximately one-quarter have no prior history of epilepsy, neurological abnormality or developmental delay prior to the onset of LGS symptoms. Underlying pathologies causing LGS may include encephalitis and/or meningitis, brain malformations (e.g., cortical dysplasias), birth injury, hypoxia-ischemia injury, frontal lobe lesions, and trauma.

An important differential diagnosis is 'Pseudo-Lennox-Syndrome', also called atypical benign partial epilepsy of childhood, which differs from LGS, in that there are no tonic seizures; sleeping EEG provides the best basis for distinguishing between the two. In addition, 'Pseudo-Lennox-Syndrome' has an entirely different etiology and prognosis than LGS.

### Treatment

The optimal treatment for Lennox-Gastaut syndrome has yet to be established. Many different medicines and therapies have been tried in the past, and some are still used currently in the treatment of this disorder, with varying success. For example: Lamotrigine, Felbamate, Rufinamide, Clobazam, Clonazepam, Topiramate are approved in US and EU. Nitrazepam and Valproate are approved in the EU (and used in the US), and Zonisamide and benzodiazepines are used (though not currently approved) in the US and EU. Mpm-pharmacologics used in the US and EU include vagus nerve stimulation, ketogenic diet and surgery.

A variety of therapeutic approaches are currently used in LGS, including conventional antiepileptic medications, diet and surgery, however the evidence supporting these therapies is not robust and treatment remains most often ineffective. The use of several common first-line treatments is based on clinical experience or conventional wisdom; examples include broad spectrum anti-convulsant medications, such as valproic acid, and benzodiazepines, most often clonazepam and clobazam. A few drugs have been proven effective for some patients for certain seizure types by double-blind placebo-controlled studies; examples include clobazam, lamotrigine, topiramate, felbamate, and rufinamide, although most patients continue to have significant seizures even while taking these medications. Second-line medications currently in use, such as zonisamide, are prescribed based on results of some open-label uncontrolled studies. The ketogenic diet may be useful in some patients with LGS refractory to medical treatment. Surgical options for LGS include corpus callostomy (for drop attacks), vagus nerve stimulation, and focal cortical resection (in the presence of a single resectable lesion). However, it should be noted that significant improvement from any of these therapies alone or in combination is a rare occurrence.

Despite the severity of LGS's symptoms and the frequency with which it occurs (it accounts for up to 10% of all childhood epilepsies), there is currently no standard evidence-based treatment for the disease. A comprehensive review of the literature [*see* Hancock EC & Cross JH, Treatment of Lennox-Gastaut syndrome (Review), published in The Cochrane Library 2013, Issue 2] discovered only nine randomized controlled trials which evaluated the pharmaceutical treatment of the syndrome. The authors concluded that there is a paucity of research and "... that no monotherapy (to date) has been shown to be highly effective in this syndrome." *Id* at page 12. The authors further concluded that "[t]he optimum treatment for LGS remains uncertain and no study to date has shown any one drug to be highly efficacious". *Id* at page 12.

Without being bound by theory, fenfluramine has been known to trigger the release of serotonin (5-HT) in the brain due to disruption of its vesicular storage and to inhibit serotonin reuptake. Fenfluramine's mechanism of action made it suitable for the treatment of epilepsy. In fact, there are no scientific publications demonstrating or even hypothesizing that 5-HT abnormalities are a possible underlying pathophysiologic cause for LGS or are causally related to the associated seizures in this specific epilepsy condition. Furthermore, since there has been no scientific hypothesis relating serotonin abnormalities in LGS, there are no studies nor even individual case reports in the medical literature which describe attempts to treat LGS using medications that interacts with serotonin. The lack of data or even speculation in the literature regarding the use of fenfluramine or serotonergic agents in general to treat LGS are facts that strongly support the unexpected nature of this invention: given that LGS is a devastating refractory epilepsy condition and the number of people affected, investigators would be strongly motivated to investigate any treatment they perceived as having any potential for efficacy.

Thus, according to the present disclosure, provided herein are method of treating epilepsy by stimulating one or more 5-HT receptors in the brain of a patient by administering an effective dose of fenfluramine to said patient, said one or more 5-HT receptors being selected from one or more of 5-HT₁, 5-HT_{1A}, 5-HT_{1B}, 5-HT_{1C}, 5-HT_{1D}, 5-HT_{1E}, 5-HT_{1F}, 5-HT₂, 5-HT_{2A}, 5-HT_{2B}, 5-HT_{2C}, 5-HT₃, 5-HT₄, 5-HT₅, 5-HT_{5A}, 5-HT_{5B} 5-HT₆, and 5-HT₇ amongst others. In certain embodiments of this aspect of the invention, the patient has been diagnosed with epilepsy.

In some embodiments, fenfluramine may be used in the treatment of epilepsy, particularly patients having Dravet syndrome, Lennox-Gastault syndrome or other forms of epileptic encephalopathy, wherein the patients also have a disease or disorder associated with reduced cognitive function.

Cognitive impairment may be observed in patients suffering from symptoms of epilepsy, such as a disruption of both basic cognitive functions and "higher level" executive function. Executive function traditionally encompasses the cognitive abilities of working memory, self-regulation, inhibitory control and attentional control, and is sometimes assessed using lab-based performance measures, or using tests such as the Behavior Rating Inventory of Executive Function (BRIEF). It has been discovered and described herein that treatment with fenfluramine can improve cognitive function (e.g., as measured by BRIEF).

The present disclosure provides methods and compositions for improving patients' cognitive function (e.g., as measured by BRIEF, Wechsler memory scale, MOCA, EXAMINER or other scale), particularly in children and young adults. In some embodiments, the patient is also being treated for an epileptic disease or disorder, e.g., Dravet syndrome and/or Lennox-Gastaut syndrome. The present disclosure helps in meeting that need, as it relates to the discovery that administration of fenfluramine over a period of time is associated with improvement in cognitive function, as measured, for example, by BRIEF, Wechsler memory scale, MOCA, EXAMINER or other scale(s). As set forth herein, it has been discovered that administration of fenfluramine can be beneficial in treating diseases and disorders affecting cognitive function.

A specific aspect of the invention includes treating a pediatric (including adolescents up to and including age 18) patient with fenfluramine to improve the child's or adolescent's performance on the BRIEF or other measure of cognitive function in everyday life, at school, at work or at home.

Another specific aspect of the invention includes treating an adult (older than age 18) patient with fenfluramine to improve the adult's performance on the BRIEF, Wechsler memory scale, MOCA, EXAMINER or other measure of cognitive function in everyday life, at school, at work or at home.

An aspect of the present disclosure includes a method of improving, and/or measuring improvement in, cognitive function in a patient, comprising administering fenfluramine, or a pharmaceutically acceptable salt thereof, to a patient. In some embodiments, the fenfluramine may be administered for a period of months or years (e.g., one, two three, 6, 9, 12, 15, 18, 21, etc. months, up to an including 3 years, for example). The measure of cognitive function may include administering the BRIEF test, Wechsler memory scale, MOCA, EXAMINER or other scale to the patient both before and after fenfluramine treatment (for example, every three months, every six months, or yearly, after the start of, and continuing over the course of, fenfluramine treatment), as a qualitative and quantitative measure of improvement in cognitive function.

In some embodiments, the patient has been diagnosed with a disease or condition selected from an epilepsy or epileptic encephalopathy (e.g., Dravet syndrome, Doose syndrome, infantile spasms, Lennox-Gastaut syndrome); attentional disorders (e.g., attention deficit disorder (ADD) or attention deficit/hyperactivity disorder (ADHD)); developmental disorders, such as autism spectrum disorders (ASDs), including autism, Asperger syndrome, pervasive developmental disorder (PDD) not otherwise specified (PDD-NOS); oppositional defiant disorder (ODD); learning disabilities (e.g. dyslexia, dyscalculia); Tourette syndrome; traumatic brain injury; lead exposure; anxiety and/or depression; and low birth weight, or any combination thereof.

In some embodiments, the patient has been diagnosed with Dravet syndrome.

In some embodiments, the patient has been diagnosed with epileptic encephalopathy.

In some embodiments, a symptom of the epileptic encephalopathy is seizure, and wherein the fenfluramine is formulated with a pharmaceutically acceptable carrier and an effective dose is less than 10.0 mg/kg/day, or less than 1.0 mg/kg/day, or approximately 0.8 mg/kg/day, or approximately 0.5 mg/kg/day, or approximately 0.2 mg/kg/day, or approximately 0.01 mg/kg/day.

In some embodiments, the fenfluramine is administered in a dosage form selected from the group consisting of oral, injectable, transdermal, inhaled, nasal, rectal, vaginal and parenteral delivery.

In some embodiments, the dosage form is an oral composition in an amount selected from the group consisting of 30 mg/day or less, 20 mg/day or less, 10 mg/day or less and 5 mg/day or less. In some embodiments, the oral composition is a solution.

In some embodiments of this method, fenfluramine is the only pharmaceutically active ingredient administered to the patient.

In some embodiments of this method, fenfluramine is used as an adjunctive therapy in a patient. In some embodiments of this method, fenfluramine is used as an adjunctive therapy in a patient with epilepsy or epileptic encephalopathy. In some embodiments of this method, fenfluramine is used as an adjunctive therapy in a patient with Dravet syndrome or Lennox-Gastault syndrome (LGS).

In some embodiments, at least one co-therapeutic agent is administered, and wherein said agent is selected from the group consisting of Brivaracetam, bromides (e.g., Potassium Bromide, Sodium Bromide), Cannabidiol, Carbamazepine, Clonidine, Ergenyl Chrono, Ethosuximide, Felbamate, Fosphenytoin, Lacosamide, Lamotrigine, Levetiracetam, Levocarnitine, Mesuximide, Nitrazepam, Oxcarbazepine, Perampanel, Phenobarbital, Pregabalin, Progabide, Pyridoxine, Rufinamide, Sultiame, Tizanidine, Topiramate, Stiripentol, Valproate semisodium, Valproate sodium, Valproic acid, Verapamil, Zonisamide, and benzodiazepines such as Clobazam, Clonazepam, Diazepam, Ethyl Loflazepate, Lorazepam and Midazolam, and a pharmaceutically acceptable salt or base thereof.

In some embodiments, the fenfluramine treatment continues in amounts and over a period of time so as to improve cognitive function as assessed via the BRIEF, Wechsler memory scale, MOCA, EXAMINER or other clinical and/or index score(s). In some embodiments, the patient's score on a test of cognitive function is improved by a statistically significant percentage. In some embodiments, the patient's clinical global impression (CGI-I rating) is improved. In some embodiments, at least one parameter of the patient's score(s) is improved by 5% or more, 10% or more, 15% or more, 25% or more, 50% or more, or 75% or more, or by 1 or more levels on the rating scale.

In some embodiments, the patient is also being treated for epilepsy or epileptic encephalopathy.

In some embodiments, the patient diagnosed with epilepsy is 18 years of age or younger. In some embodiments, the patient diagnosed with epilepsy is an adult over 18 years of age.

Another aspect of the present disclosure includes a kit, comprising a fenfluramine formulation, a package, and a package insert comprising instructions for use in improving cognitive function in a patient.

Another aspect of the present disclosure includes a kit, comprising a container comprising a plurality of doses of a formulation comprising a pharmaceutically acceptable carrier and an active ingredient comprising fenfluramine; and instructions for treating the patient with the formulation and assessing the patient's cognitive function (e.g., by obtaining a score on the BRIEF, Wechsler memory scale, MOCA, EXAMINER or other scale) before and after treatment with the formulation.

DOSE BY WEIGHT (MG/KG/DAY): In embodiments of the invention, any effective dose of fenfluramine can be employed. However, surprisingly low doses of fenfluramine have been found by the inventors to be effective, particularly for inhibiting or eliminating seizures in epilepsy patients. In some cases, in a preferred embodiment of the invention, a daily dose of less than about 10 mg/kg/day, such as less than about 9 mg/kg/day, less than about 8 mg/kg/day, less than about 7 mg/kg/day, less than about 6 mg/kg/day, less than about 5 mg/kg/day, less than about 4 mg/kg/day, less than about 3.0 mg/kg/day, less than about 2.5 mg/kg/day, less than about 2.0 mg/kg/day, less than about 1.5 mg/kg/day, less than about 1.0 mg/kg/day, such as about 0.95 mg/kg/day, about 0.9 mg/kg/day, about 0.85 mg/kg/day, about 0.8 mg/kg/day, about 0.75 mg/kg/day, about 0.7 mg/kg/day, about 0.65 mg/kg/day, about 0.6 mg/kg/day, about 0.55 mg/kg/day, about 0.5 mg/kg/day, about 0.45 mg/kg/day, about 0.4 mg/kg/day, about 0.350 mg/kg/day, about 0.3 mg/kg/day, about 0.25 mg/kg/day, about 0.2 mg/kg/day, about 0.15 mg/kg/day to about 0.1 mg/kg/day, about 0.075 mg/kg/day, about 0.05 mg/kg/day, about 0.025 mg/kg/day, about 0.0225 mg/kg/day, about 0.02 mg/kg/day, about 0.0175 mg/kg/day, about 0.015 mg/kg/day, about 0.0125 mg/kg/day, or about 0.01 mg/kg/day is employed.

Put differently, a preferred dose is less than about 10 to about 0.01 mg/kg/day. In some cases the dose is less than about 10.0 mg/kg/day to about 0.01 mg/kg/day, such as less than about 5.0 mg/kg/day to about 0.01 mg/kg/day, less than about 4.5 mg/kg/day to about 0.01 mg/kg/day, less than about 4.0 mg/kg/day to about 0.01 mg/kg/day, less than about 3.5 mg/kg/day to about 0.01 mg/kg/day, less than about 3.0 mg/kg/day to about 0.01 mg/kg/day, less than about 2.5 mg/kg/day to about 0.01 mg/kg/day, less than about 2.0 mg/kg/day to about 0.01 mg/kg/day, less than about 1.5 mg/kg/day to about 0.01 mg/kg/day, or less than about 1.0 mg/kg/day to 0.01 mg/kg/day, such as less than about 0.9 mg/kg/day, less than about 0.8 mg/kg/day, less than about less than about 0.7 mg/kg/day, less than about 0.6 mg/kg/day to about 0.01 mg/kg/day, less than about 0.5 mg/kg/day to about 0.01 mg/kg/day, less than about 0.4 mg/kg/day to about 0.01 mg/kg/day, less than about 0.3 mg/kg/day to about 0.01 mg/kg/day, or less than about.0.2 mg/kg/day to about 0.01 mg/kg/day.

As indicated above the dosing is based on the weight of the patient. However, for convenience the dosing amounts may be preset such as in the amount of 1.0 mg, 2.5 mg, 5 mg, 10 mg, 15 mg, 20 mg, 30 mg, 40 mg, or 50 mg. In certain instances, the dosing amount may be preset such as in the amount of about 0.25 mg to about 5 mg, such as about 0.25 mg, about 0.5 mg, about 0.75 mg, about 1.0 mg, about 1.25 mg, about 1.5 mg, about 1.75 mg, about 2.0 mg, about 2.25 mg, about 2.5 mg, about 2.75 mg, about 3.0 mg, about 3.25 mg, about 3.5 mg, about 3.75 mg, about 4.0 mg, about 4.25 mg, about 4.5 mg, about 4.75 mg, or about 5.0 mg.

In general, the smallest dose which is effective should be used for the particular patient.

The dosing amounts described herein may be administered one or more times daily to provide for a daily dosing amount, such as once daily, twice daily, three times daily, or four or more times daily, etc.

In certain embodiments, the dosing amount is a daily dose of 30 mg or less, such as about 30 mg, about 29 mg, about 28 mg, about 27 mg, about 26 mg, about 25 mg, about 24 mg, about 23 mg, about 22 mg, about 21 mg, about 20 mg, about 19 mg, about 18 mg, about 17 mg, about 16 mg, about 15 mg, about 14 mg, about 13 mg, about 12 mg, about 11 mg, about 10 mg, about 9 mg, about 8 mg, about 7 mg, about 6 mg, about 5 mg, about 4 mg, about 3 mg, about 2 mg, or about 1 mg. In general the smallest dose which is effective should be used for the particular patient. In some cases, the dose is generally well below the dosing used in weight loss.

ROUTES OF ADMINISTRATION: The dose of fenfluramine administered according to the methods of the present invention can be administered systemically or locally. Methods of administration may include administration via enteral routes, such as oral, buccal, sublingual, and rectal; topical administration, such as transdermal and intradermal; and parenteral administration. Suitable parenteral routes include injection via a hypodermic needle or catheter, for example, intravenous, intramuscular, subcutaneous, intradermal, intraperitoneal, intraarterial, intraventricular, intrathecal, and intracameral injection and non-injection routes, such as intravaginal rectal, or nasal administration. In certain embodiments, it may be desirable to administer one or more compounds of the invention locally to the area in need of treatment. This may be achieved, for example, by local infusion during, topical application, by injection, by means of a catheter, by means of a suppository, or by means of an implant, said implant being of a porous, non-porous, or gelatinous material, including membranes, such as sialastic membranes, or fibers.

DOSAGE FORMS / ROUTE OF ADMINISTRATION: The dose of fenfluramine administered in the methods of the present invention can be formulated in any pharmaceutically acceptable dosage form including, but not limited to (a) oral dosage forms such as tablets including orally disintegrating tablets, capsules, and lozenges, oral solutions or syrups, oral emulsions, oral gels, oral films, buccal liquids, powder e.g. for suspension, and the like; (b) injectable dosage forms; (c) transdermal dosage forms such as transdermal patches, ointments, creams; (c) inhaled dosage forms; and/or (e) nasally, (f) rectally, (g) vaginally administered dosage forms.

DOSAGE FORM / FREQUENCY OF ADMINISTRATION: Such dosage forms can be formulated for once a day administration, or for multiple daily administrations (e.g. 2, 3 or 4 times a day administration). Alternatively, for convenience, dosage forms can be formulated for less frequent administration (e.g., monthly, bi-weekly, weekly, every fourth day, every third day, or every second day), and formulations which facilitate extended release are known in the art.

DOSAGE FORMS / PREPARATION, COMPONENTS: The dosage form of fenfluramine employed in the methods of the present invention can be prepared by combining fenfluramine or a pharmaceutically acceptable salt thereof with one or more pharmaceutically acceptable diluents, carriers, adjuvants, and the like in a manner known to those skilled in the art of pharmaceutical formulation.

ORAL DOSAGE FORMS / SUITABLE FORMULATION TYPES & COMPONENTS THEREOF: In some embodiments, formulations suitable for oral administration can include (a) liquid solutions, such as an effective amount of the compound dissolved in diluents, such as water, or saline; (b) capsules, sachets or tablets, each containing a predetermined amount of the active ingredient (fenfluramine), as solids or granules; (c) suspensions in an appropriate liquid; and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can include the active ingredient in a flavor, usually sucrose and acacia or tragacanth, as well as pastilles including the active ingredient in an inert base, such as gelatin and glycerin, or sucrose and acacia, emulsions, gels, and the like containing, in addition to the active ingredient, such excipients as are described herein.

ORAL DOSAGE FORMS / EXCIPIENTS: For an oral solid pharmaceutical formulation, suitable excipients include pharmaceutical grades of carriers such as mannitol, lactose, glucose, sucrose, starch, cellulose, gelatin, magnesium stearate, sodium saccharine, and/or magnesium carbonate. For use in oral liquid formulations, the composition may be prepared as a solution, suspension, emulsion, or syrup, being supplied either in solid or liquid form suitable for hydration in an aqueous carrier, such as, for example, aqueous saline, aqueous dextrose, glycerol, or ethanol, preferably water or normal saline. If desired, the composition may also contain minor amounts of non-toxic auxiliary substances such as wetting agents, emulsifying agents, or buffers.

By way of illustration, the fenfluramine composition can be admixed with conventional pharmaceutically acceptable carriers and excipients *(i.e.,* vehicles) and used in the form of aqueous solutions, tablets, capsules, elixirs, suspensions, syrups, wafers, and the like. Such pharmaceutical compositions contain, in certain embodiments, from about 0.1% to about 90% by weight of the active compound, and more generally from about 1% to about 30% by weight of the active compound. The pharmaceutical compositions may contain common carriers and excipients, such as corn starch or gelatin, lactose, dextrose, sucrose, microcrystalline cellulose, kaolin, mannitol, dicalcium phosphate, sodium chloride, and alginic acid. Disintegrators commonly used in the formulations of this invention include croscarmellose, microcrystalline cellulose, corn starch, sodium starch glycolate and alginic acid.

Formulations suitable for topical administration may be presented as creams, gels, pastes, or foams, containing, in addition to the active ingredient, such carriers as are appropriate. In some embodiments the topical formulation contains one or more components selected from a structuring agent, a thickener or gelling agent, and an emollient or lubricant. Frequently employed structuring agents include long chain alcohols, such as stearyl alcohol, and glyceryl ethers or esters and oligo(ethylene oxide) ethers or esters thereof. Thickeners and gelling agents include, for example, polymers of acrylic or methacrylic acid and esters thereof, polyacrylamides, and naturally occurring thickeners such as agar, carrageenan, gelatin, and guar gum. Examples of emollients include triglyceride esters, fatty acid esters and amides, waxes such as beeswax, spermaceti, or carnauba wax, phospholipids such as lecithin, and sterols and fatty acid esters thereof. The topical formulations may further include other components, e.g., astringents, fragrances, pigments, skin penetration enhancing agents, sunscreens (e.g., sunblocking agents), etc.

Particular formulations of the invention are in an oral liquid form. The liquid can be a solution or suspension and may be an oral solution or syrup, which is included in a bottle with a syringe graduated in terms of milligram amounts which will be obtained in a given volume of solution. The liquid solution makes it possible to adjust the volume of solution for appropriate dosing of small children, who can be administered fenfluramine in an amount anywhere from 1.25 mg to 30 mg and any amount between in 0.25 milligram, increments and thus administered in amounts of 1.25 mg, 1.5 mg, 1.75 mg, 2.0 mg, etc.

Fenfluramine may be co-administered with other known pharmaceutical drugs such as a co-therapeutic agent selected from the group consisting of Brivaracetam, bromides (e.g., Potassium Bromide, Sodium Bromide), Cannabidiol, Carbamazepine, Clonidine, Ergenyl Chrono, Ethosuximide, Felbamate, Fosphenytoin, Lacosamide, Lamotrigine, Levetiracetam, Levocarnitine, Mesuximide, Nitrazepam, Oxcarbazepine, Perampanel, Phenobarbital, Pregabalin, Progabide, Pyridoxine, Rufinamide, Sultiame, Tizanidine, Topiramate, Stiripentol, Valproate semisodium, Valproate sodium, Valproic acid, Verapamil, Zonisamide, and benzodiazepines such as Clobazam, Clonazepam, Diazepam, Ethyl Loflazepate, Lorazepam and Midazolam, and a pharmaceutically acceptable salt or base thereof.

The co-therapeutic agents have recommended dosing amounts. Those recommended dosing amounts are provided within the most current version of the Physician's Desk Reference (PDR) or online at emedicine.medscape.com.

In connection with the present invention, the co-therapeutic agent can be used in the recommended dosing amount or can be used in a range of from 100^{th} to 100 times 1/10 to 10 times 1/5 to 5 times 1/2 to twice the recommended dosing amount or any incremental 1/10 amount in between those ranges.

As a specific example of a combination of co-therapeutic agents with fenfluramine, the co-therapeutic agent may be any one of, any two of, or all three of stiripentol, clobazam, and valproate. The fenfluramine may be administered in a daily amount of 0.8 mg/kg of patient body weight. The fenfluramine may be administered in a daily amount of 0.5 mg/kg of patient body weight when co-administered with 50 mg/kg/day of stiripentol, with or without 20 mg of clobazam, and/or 25 mg per kg of valproate. Each of those amounts may be increased to twice, three times, five times, or ten times that amount or decreased by 10%, 50%, or 75%.

In alternate embodiments, the dispensing device may be a syringe or graduated pipette useful for delivering varying doses of the fenfluramine liquid. In another embodiment, the dispensing device is a metered dosing device capable of dispensing a fixed volume of fenfluramine liquid. In one exemplary embodiment, the dose delivered by the metered dosing device is adjustable.

The formulation may be a solution or suspension and is prepared such that a given volume of the formulation contains a known amount of active fenfluramine.

For example, in one embodiment of this aspect, the dispensing device is a syringe is graduated in one milliliter increments and the liquid fenfluramine formulation is characterized such that one milliliter in volume of formulation includes precisely one milligram of fenfluramine. In this manner, the patient may be correctly dosed with a desired milligram dosage of fenfluramine based on a volume of liquid formulation administered to the patient orally.

In alternate embodiments, the dispenser is a syringe connected to the container and configured to withdraw the liquid formulation from the container, wherein the syringe is marked with levels of graduation noting volume of formulation withdrawn, or a metered dose dispenser for delivering a predetermined volume of the formulation to said patient, or a metered dispensing device calibrated to deliver a predetermined volume of the liquid, permitting convenient, consistent, and accurate dosing.

In some embodiments of this method, fenfluramine is the only pharmaceutically active ingredient administered to the patient.

In some embodiments of this method, fenfluramine is used as an adjunctive therapy in a patient. In some embodiments of this method, fenfluramine is used as an adjunctive therapy in a patient with epilepsy or epileptic encephalopathy. In some embodiments of this method, fenfluramine is used as an adjunctive therapy in a patient with Dravet syndrome or Lennox-Gastault syndrome (LGS).

In a method of the present invention, fenfluramine can be employed as a co-therapy in the treatment of epilepsy. Fenfluramine can be co-administered in combination with one or more pharmaceutically active agents, which may be provided together with the fenfluramine in a single dosage formulation, or separately, in one or more separate pharmaceutical dosage formulations. Where separate dosage formulations are used, the subject composition and ore or more additional agents can be administered concurrently, or at separately staggered times, *i.e.,* sequentially.

In one embodiment, the agents are co-therapeutic agents, such as anticonvulsants. Suitable co-therapeutic agents can be selected from the group consisting of Brivaracetam, bromides (e.g., Potassium Bromide, Sodium Bromide), Cannabidiol, Carbamazepine, Clonidine, Ergenyl Chrono, Ethosuximide, Felbamate, Fosphenytoin, Lacosamide, Lamotrigine, Levetiracetam, Levocarnitine, Mesuximide, Nitrazepam, Oxcarbamazepine, Perampanel, Phenobarbital, Pregabalin, Progabide, Pyridoxine, Rufinamide, Sultiame, Tizanidine, Topiramate, Stiripentol, Valproate semisodium, Valproate sodium, Valproic acid, Verapamil, Zonisamide, and benzodiazepines such as Clobazam, Clonazepam, Diazepam, Ethyl Loflazepate, Lorazepam and Midazolam. Use of a pharmaceutically acceptable salt of a co-therapeutic agent is also contemplated.

In some embodiments, the subject/patient may have been previously treated with a medication, prior to treatment with fenfluramine, wherein the prior agent is selected from Acetazolamide, Brivaracetam, Carbamazepine, Clobazam, Clonazepam, Diazepam, Ergenyl Chrono, Ethosuximide, Felbamate, Gabapentin, Lacosamide, Lamotrigine, Levetiracetam, Lorazepam, Mesuximide, Oxcarbazepine, Perampanel, Phenobarbital, Phenytoin, Phenytoin sodium, Pregabalin, Rufinamide, Stiripentol, Sultiame, Topiramate, Valproate semisodium, Valproate sodium, Valproic acid, Vigabatrin, Zonisamide, and a pharmaceutically acceptable salt or base of any of these.

Fenfluramine can be administered in the form of the free base, or in the form of a pharmaceutically acceptable salt, for example selected from the group consisting of hydrochloride, hydrobromide, hydroiodide, maleate, sulphate, tartrate, acetate, citrate, tosylate, succinate, mesylate and besylate. Further illustrative pharmaceutically acceptable salts can be found in Berge et al., J. Pharm Sci. (1977) 68(1): 1-19.

Fenfluramine for use in the methods of the present invention may be produced according to any pharmaceutically acceptable process known to those skilled in the art. Examples of processes for synthesizing fenfluramine are provided in the following documents: GB1413070, GB1413078 and EP441160.

The dose of fenfluramine to be used in a method of the present invention can be provided in the form of a kit, including instructions for using the dose in one or more of the methods of the present invention. In certain embodiments, the kit can additionally comprise a dosage form comprising one or more co-therapeutic agents.

A method of the present invention can be practiced on any appropriately diagnosed patient. In alternate exemplary embodiments of the present invention, the patient is aged about 18 or less, about 16 or less, about 14 or less, about 12 or less, about 10 or less, about 8 or less, about 6 or less or about 4 or less to about 0 months or more, about 1 month or more, about 2 months or more, about 4 months or more, about 6 months or more or about 1 year or more. Thus, in some embodiments, the diagnosed patient is about one month old to about 18 years old when treated.

In some embodiments, the patient is an adult over 18 years of age.

The invention is further illustrated in the following Examples.

### EXAMPLE 1

### THERAPY WITH FENFLURAMINE AND TESTING FOR IMPROVED COGNITIVE FUNCTION AS ASSESSED BY BRIEF - DRAVET SYNDROME

Catarino *et al.* reported the results of a retrospective study of 22 adult patients with Dravet syndrome, and found three of the patients who had experienced improvement in seizure control after being switched to appropriate anti-epileptic drug (AED), as well as improvements in cognitive function (Catarino CB, Liu JY, Liagkouras I, et al., 2011, Brain 134:2982-3010). Furthermore, in a recently published trial of cannabidiol in Dravet syndrome the active treatment groups did not achieve significant difference from placebo on cognitive function (Devinsky, NEJM 2017). To date, no prospective placebo-controlled trial of AEDs in Dravet syndrome (or any other epileptic encephalopathy) has been able to demonstrate such benefits; the present disclosure provides the first demonstration of an improvement in cognitive function in a prospective randomized controlled trial of fenfluramine treatment of pediatric Dravet patients.

Without being limited by theory, and for the present study, it was hypothesized that, in children and adults with epileptic encephalopathies, cognitive impairment may be caused by seizures, by an underlying genetic abnormality, or a combination of both. Historically, for example, upon fenfluramine treatment, a significant reduction in monthly convulsive seizure frequency (MCSF) may contribute to improvement in cognitive function (Brunklaus A, Zuberi SM. "Dravet syndrome--from epileptic encephalopathy to channelopathy." Epilepsia 2014, 55:979-84; Catarino et al., "Dravet syndrome as epileptic encephalopathy: evidence from long-term course and neuropathology." Brain 2011, 134:2982-3010). However, given that improvements in cognitive function have not been reported in previous placebo controlled trials, fenfluramine's ability to improve cognitive function is likely not limited to its effects in reducing seizure frequency, and is likely a complex interaction of the reduction in MCSF as well as other factors, including genetics. A direct mechanism of action has not been ruled out.

For the present disclosure, a study was conducted in which a fenfluramine HCl oral solution was used as an adjunctive therapy in children and young adults with Dravet syndrome for 22 weeks, with a follow-up 3 to 6 months after the last dose of study medication for final safety monitoring.

The results for the first 119 consecutive subjects (out of an anticipated total of 240 subjects) who enrolled and were randomized in either trial before a prespecified cut-off date are presented here as "Study 1."

### Inclusion and Exclusion criteria

Human patients, age 2 to 18 years, inclusive, having a documented medical history to support a clinical diagnosis of Dravet syndrome and a history of seizures (either generalized tonic-clonic or unilateral clonic or bilateral clonic, and prolonged) were recruited, and selected for inclusion in the study according to criteria comprising a combination of age, physical and psychological characteristics, and (optionally) resistance to treatment with conventional therapies. Included subjects were assessed over the course of this 22-week study. The study protocols were reviewed and approved by the institutional review board or ethics committee for each study site before any study activation at the site. All patients or their legal representatives signed informed consent/assent prior to enrolling in the trial.

In some patients, prolonged exposure to warm temperatures induced seizures and/or seizures were associated with fevers due to illness or vaccines, hot baths, high levels of activity and sudden temperature changes and/or seizures are induced by strong natural and/or fluorescent lighting, as well as certain visual patterns.

Subjects had ≥4 convulsive seizures (tonic, tonic atonic, tonic-clonic, clonic) per 4-week period for past 12 weeks prior to screening, by parent/guardian report to investigator or investigator medical notes.

All medications or interventions for epilepsy (including ketogenic diet [KD] and vagal nerve stimulation [VNS]) were stable for at least 4 weeks prior to screening and were expected to remain stable throughout the study.

Subject's parent/caregiver was willing and able to be compliant with diary completion, visit schedule and study drug accountability.

Subjects were excluded if known to: be hypersensitive to fenfluramine or any of the excipients in the study medication; have a history of pulmonary arterial hypertension; have a current or past history of cardiovascular or cerebrovascular disease (e.g., cardiac valvulopathy, myocardial infarction or stroke); be currently treated with centrally-acting anorectic agents, monoamine-oxidase inhibitors, or any centrally-acting agent with a serotonin agonist or antagonist properties, treatment with stiripentol within 21 days prior to screening, or have a positive urine test for tetrahydrocannabinol or whole blood test for cannabidiol at screening. Eligibility for enrollment was approved by the Epilepsy Study Consortium (http://epilepsyconsortium.org/).

Potential subjects enrolled in a 6-week baseline to establish seizure frequency and determine eligibility. Seizures were documented by parents or caregivers in an electronic study diary as to date, time of day, duration, and seizure type. To qualify for randomization each subject must have had ≥6 convulsive seizures during the baseline period with ≥2 in the first 3 weeks and ≥2 in the last 3 weeks. For this study convulsive seizures were defined as hemiclonic, tonic, clonic, tonic-atonic, generalized tonic-clonic, and focal with clear observable motor signs. The six-week Baseline Period started with a screening visit followed by an observation period where subjects were assessed for baseline seizure activity based on recordings of daily seizure activity entered into the diary. At the end of the Baseline period, eligible patients were randomized 1:1:1 to placebo, in a double-blind to receive the first of doses of fenfluramine HCl ("ZX008" buffered to pH 5) (0.2 mg/kg/day, 0.8 mg/kg/day, not exceeding a dose of 30 mg/day).

Randomization was double-blind and stratified by age group (<6 years, ≥6 years) across treatment groups. A placebo solution, identical in appearance and taste, was also provided. Daily doses were administered with food in 2 equal doses, one in the morning and one in the evening approximately 12 hours apart. During the first 2 weeks (the Titration Period) patients in the fenfluramine 0.8 mg/kg/day group were blindly titrated to their final dose starting with 0.2 mg/kg/day for 4 days, 0.4 mg/kg/day for 4 days, and then reaching the final dose. The other groups underwent dummy titrations to maintain the blind. Patients were maintained on their final dose for an additional 12 weeks (the Maintenance Period). At the conclusion of the Treatment Period (Titration plus Maintenance), eligible patients electing to continue in an optional open-label extension (OLE) study underwent a 2-week transition period, whereas patients exiting the study underwent a 2-week taper and follow-up.

ZX008 was supplied as an oral solution in concentrations of 1.25, 2.5, and 5 mg/mL. Subjects will be randomized to receive 1 of 2 doses of ZX008 (0.2 mg/kg/day, 0.8 mg/kg/day; up to 30 mg/day) or placebo. Study medication will be administered twice a day (BID) in equally divided doses with food. To the placebo group, as a negative control, an oral solution not containing fenfluramine was administered.

In some embodiments, the BRIEF, BRIEF-A or BRIEF-P (according to the age of the patient), is administered to establish a baseline measure of cognitive function in a patient before treatment with fenfluramine.

The BRIEF (BRIEF-A or BRIEF-P) were administered at the time of patient randomization (on visit 3, which was one day before (study day "-1") treatment began (study day 1)); the BRIEF was administered again during visit 8 (study day 43), and at the end of the study (visit 12, study day 99) to assess cognitive function. Initially, the BRIEF was included as a safety measure to assess if treatment resulted in any negative effects on cognitive function. Surprisingly, however, in addition to no negative effects on cognitive function, it was further unexpectedly observed that instead, a statistically significant improvement was observed in some of the executive function index scores on the BRIEF, while the placebo-treated group worsened on all three indexes *(i.e.,* the Behavioral Regulation Index (BRI), Metacognition Index (MI) and the Global Executive Composite Score (GEC)). Compared to baseline, randomized subjects receiving fenfluramine HCl ("ZX008") 0.8 mg/kg/day were observed to have a statistically significant improvement on both the BRIEF BRI (P < 0.05) and BRIEF GEC (P < 0.05) indices as compared to placebo, and subjects in the ZX008 0.2 mg/kg/day group were observed to have a significant improvement on the BRI (P<0.05).

### EXAMPLE 2

### THERAPY WITH FENFLURAMINE AND TESTING FOR IMPROVED GLOBAL FUNCTION AS ASSESSED BY CGI-I - DRAVET SYNDROME

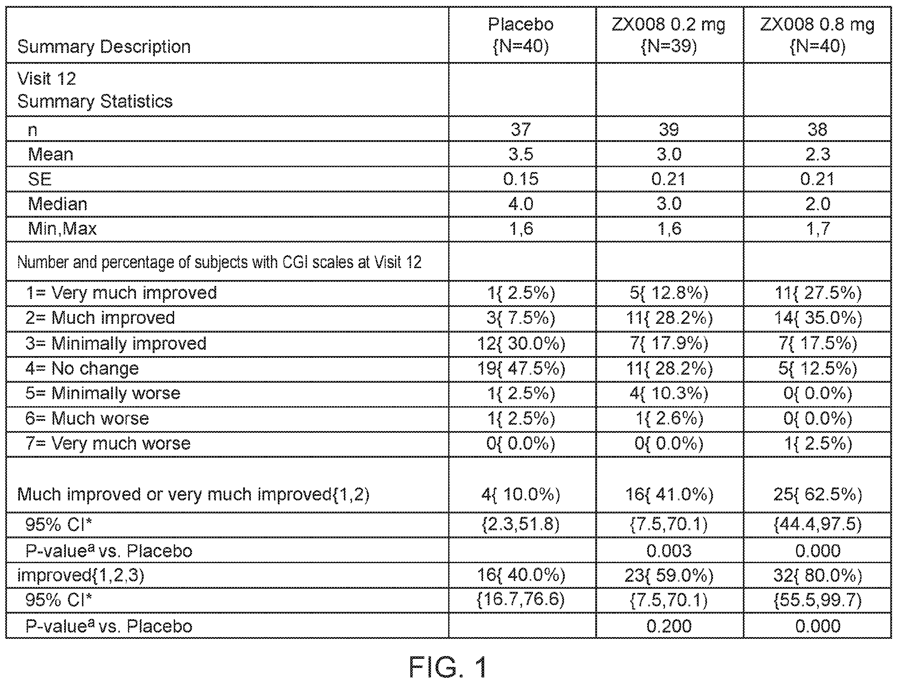

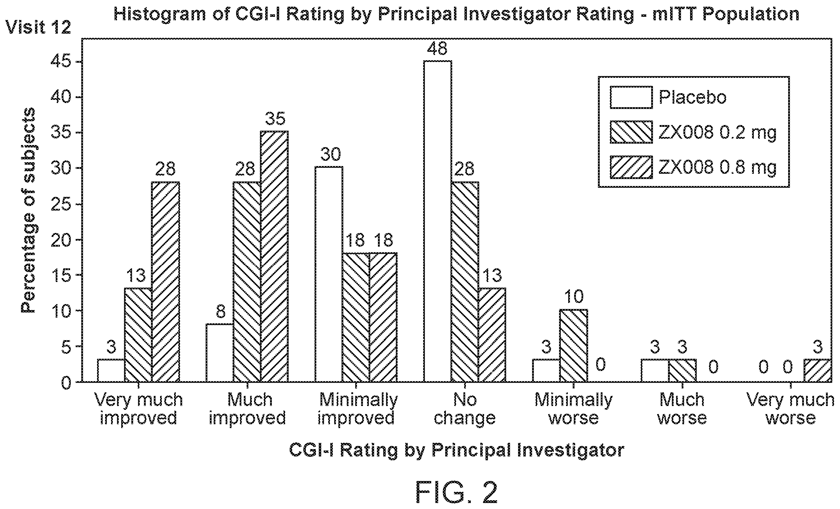

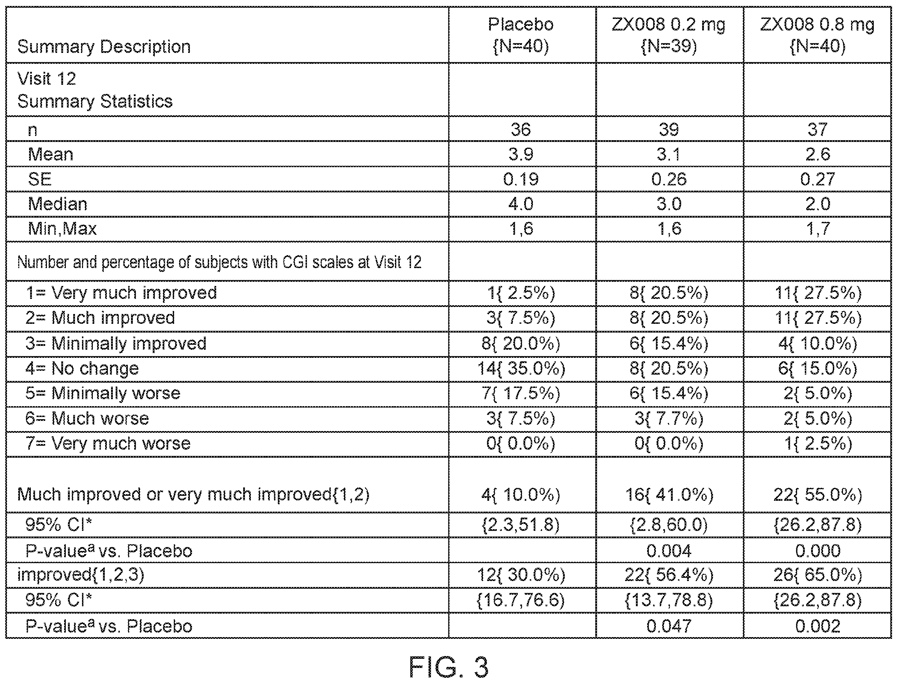

CGI-S and CGI-I ratings were made by clinical investigators and parent/caregivers in a series of phase III trials of fenfluramine. CGI changes were measured in two randomized, controlled trials with results reported herein. Study 1 was conducted as described in Example 1 and CGI ratings were made according to a set schedule of visits made to the clinic. Tables of results and bar graphs of the statistical analyses of CGI ratings for Study 1 at visit twelve (day 113) are provided in Figures 1-4. The ratings showed clinically meaningful improvements in CGI-I scores (increases in scores of Much Improved and Very Much Improved). Significantly more parents/caregivers at either 0.2 mg.kg.day or 0.8 mg.kg.day dose rated their children as "very much improved" or "much improved" than did those in the placebo group. Similar results were obtained with investigator CGI-I rating.

Study 1504 A Randomized, Double-blind, Placebo-controlled Parallel Group Evaluation of the Efficacy, Safety, and Tolerability of ZX008 as Adjunctive Antiepileptic Therapy to Stiripentol Treatment in Children and Young Adults with Dravet Syndrome also made CGI measurements during the baseline period and at specified visits.

Patient Inclusion and Dosing: A 6-week Baseline Period consisted of the establishment of initial eligibility during a screening visit followed by an observation period where subjects were assessed for baseline seizure activity based on recordings of daily seizure activity entered into a diary which established a baseline convulsive seizure frequency (CSF). Upon completion of the Baseline Period, subjects who qualified for the study were randomized (1:1) in a double-blind manner to receive ZX008 (at a dose of 0.5 mg/kg/day, maximum 20 mg/day or placebo.

Randomization was stratified by age group (≥2 to < 6 years and ≥ 6 years) to ensure balance across treatment arms. Patients were titrated to their target dose over three weeks and then remained at that fixed dose for 12 weeks. Titration occurred in 3 steps starting with a 0.2 mg/kg/day dose of ZX008 (or placebo equivalent) on Study Days 1-7, increased to a dose of 0.4 mg/kg/day on Study Day 8-14, and then increased to a dose of 0.5 mg/kg/day on Study Days 15-21; the maximum daily dose at any point was 20 mg/day. The duration of the titration period was 21 days. Following titration subjects continued treatment at their randomly assigned dose of ZX008 0.5 mg/kg/day (maximum 20 mg/day) or placebo over a 12-week Maintenance Period.

Eighty-seven patients were randomized into treatment and placebo arms, with a median age of 9 years (range, 2-19 years), across sites in Europe, the United States, and Canada. Following a six-week baseline observation period, which established a baseline CSF, patients were randomly assigned to one of two treatment groups in which ZX008 (n = 43) or placebo (n = 44) was added to their stable background regimen of stripentol plus other antiepileptic drugs. The ZX008 dose of 0.5 mg/kg/day (20 mg maximum daily dose) in this study accounted for a drug-drug interaction between stiripentol and ZX008.

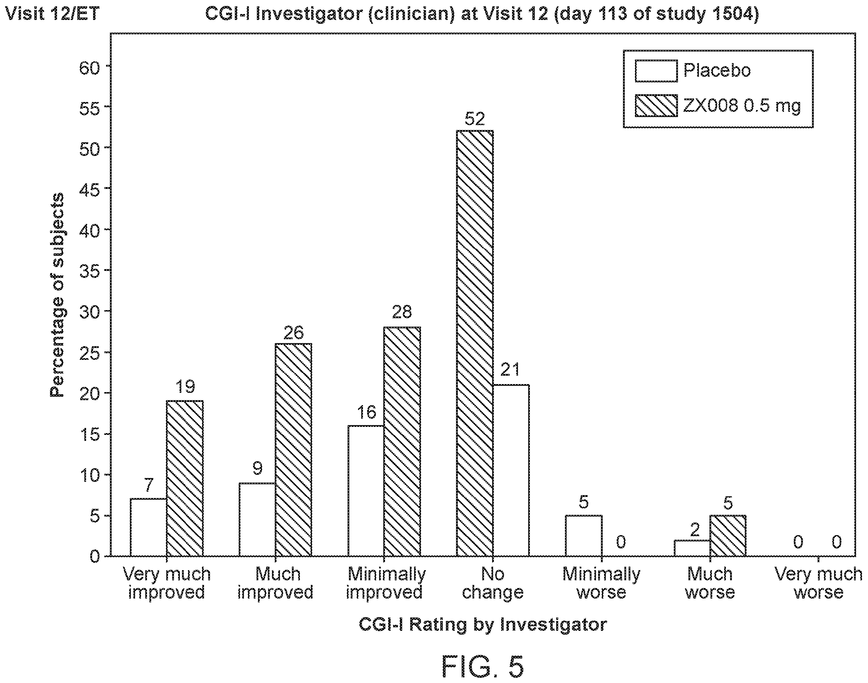

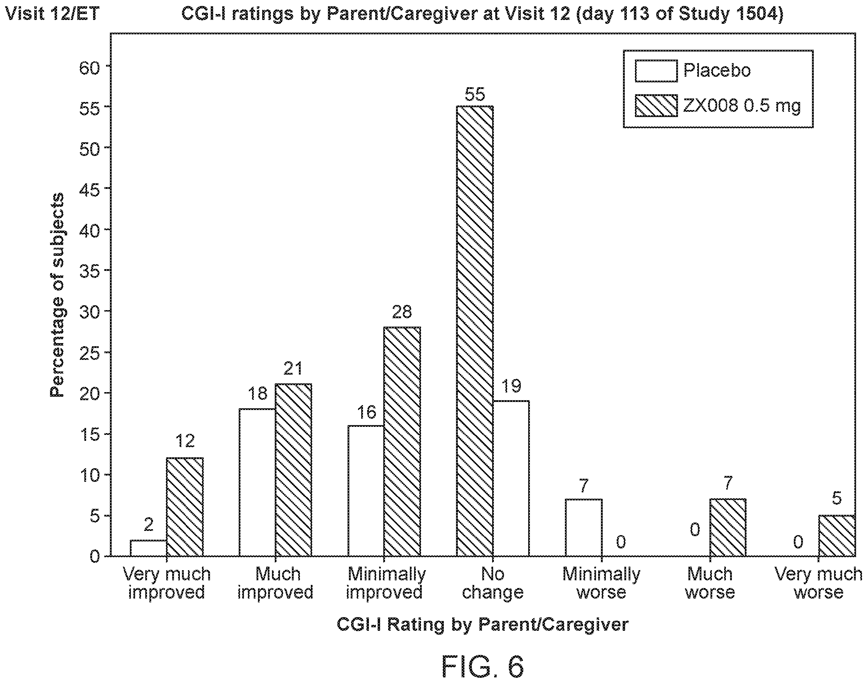

Figures 5 and 6 are graphic summaries of percentages of patients in Study 1504 showing improvements in CGI compared with patients on placebo.

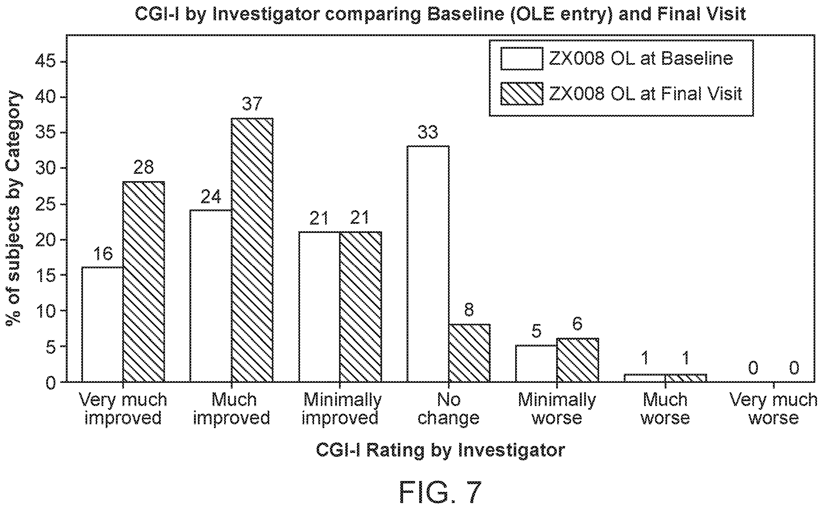

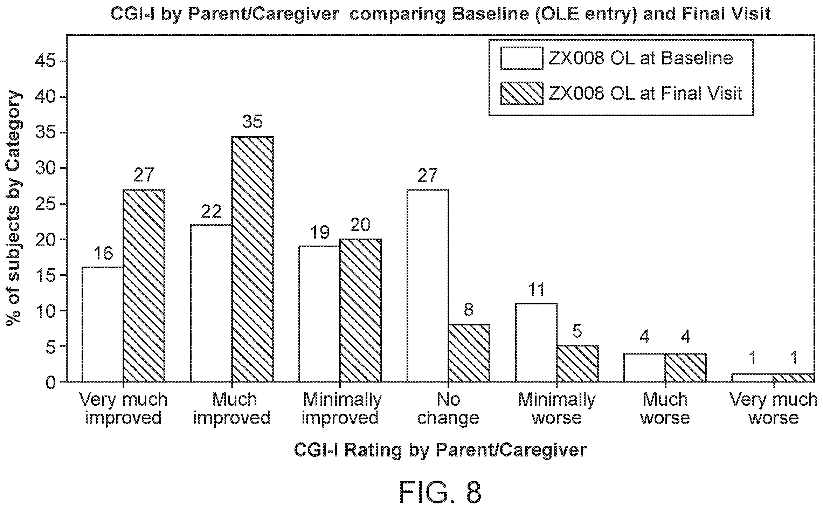

Study 1503 was an open-label, long-term safety study of ZX008 in subjects with Dravet syndrome who successfully completed 14 weeks of treatment in 1501, 1502, or 1504 Cohorts 1 and 2. Study 1503 enrolled patients all received fenfluramine treatment for up to 24 months after entering the open label phase. CGI-S (baseline scores) were determined at entry into 1503 *(i.e.,* after completing a blinded 16 week trial) and then again at each patient's final visit. Figures 7 and 8, and Tables 3 and 4 present numbers of patients having each of the 7 ratings and statistical calculations of improvements.

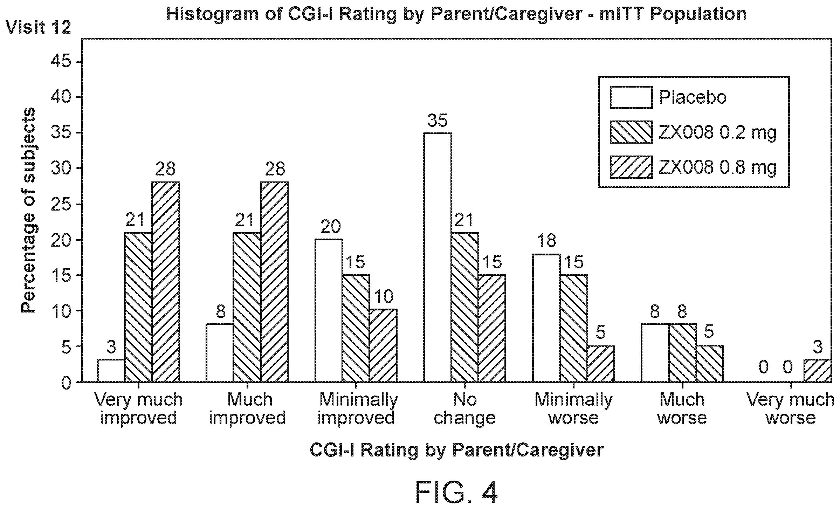

The CGI-I parent/caregiver rating at baseline, when subjects had completed participation in a core study, and the final visit is presented in Table 1 and Figure 4. At the final visit, 204 subjects (82.3%) had a CGI-I parent/caregiver rating of improved (minimally improved, much improved, or very much improved) and 155 subjects (62.5%) had a CGI-I parent/caregiver rating of much/very much improved or very much improved. The CGI-I investigator rating at baseline and the final visit is presented in 4 and Figure 8. At the final visit, 217 subjects (85.4%) had a CGI-I investigator rating of improved (minimally improved, much improved, or very much improved) and 163 subjects (64.2%) had a CGI-I investigator rating of much/very much improved. The improvements in CGI-I scores seen in the open label extension (OLE) were improvements over the score improvements seen in the core studies, demonstrating both continued improvement and durability of the treatment effect with fenfluramine.

### Safety

The incidence of treatment-emergent adverse events (TEAEs) was monitored continuously during the study. TEAEs were graded by the investigator as mild, moderate, or severe, and related or not related to study medication. Vital signs, height, weight, and clinical laboratory evaluations were performed at each study visit. The Behavior Rating Inventory of Executive Function (BRIEF) was administered at baseline and periodically during the treatment period to assess for any effects of treatment on cognitive function. Doppler echocardiography (ECHOs) and 12-lead electrocardiography (ECGs) were performed during the screening/baseline period, after 6 weeks of treatment, and after 14 weeks of treatment at the end of the maintenance period. The ECGs and ECHOs were evaluated by two independent cardiologists who were blind to treatment randomization. In the event of disagreement, a third cardiologist arbitrated the decision.

### Statistical Analysis

The standard deviation of the percentage change in monthly seizure frequency was estimated to be 55% based on results from previous randomized clinical studies of stiripentol and cannabidiol for the treatment of seizures in Dravet syndrome patients. Based on this assumption, a sample size of 40 patients per arm was determined to provide 90% power to detect a difference in mean change from baseline in monthly seizure frequency of 40 percentage points using a two-sided test at the α=0.05 significance level.

Monthly convulsive seizure frequency (MCSF) was expressed per 28 days. The primary end point was the change in mean MCSF) between the baseline period and the combined titration and maintenance periods in subjects treated with ZX008 0.8 mg/kg/day compared with the group treated with placebo. Five key secondary end points were pre-specified: the comparison of the ZX008 0.2 mg/kg/day group with placebo for the change in MCSF between baseline and the combined titration and maintenance periods, comparison of both ZX008 groups independently with placebo on the proportion of subjects who achieved a ≥50% reduction from baseline in MCSF and the comparison of both ZX008 groups with placebo on the longest seizure-free interval observed in each group. A serial gatekeeping procedure was used to maintain the simultaneous type 1 error rate at α=0.05 across the analyses of the primary and 5 key secondary end points. The primary and all key secondary end point analyses were performed on the mITT population defined as all subjects who received at least one dose of study medication and had at least 1 week of post-treatment seizure diary data.

The primary end point was analyzed using an analysis of covariance (ANCOVA) model with treatment and age group (< 6 years, ≥6 years) as factors, log baseline MCSF as a covariate and log MCSF during the combined titration and maintenance periods as the response. Estimated treatment differences and CI endpoints were exponentiated to yield an estimate of the percentage difference between groups. The comparison of ZX008 0.2 mg/kg/day with placebo for change in MCSF from baseline to the combined titration and maintenance periods was analyzed similarly. Treatment groups were compared on the proportion of patients who achieved a ≥50% reduction in MCSF using a logistic regression model that incorporated the same factors as the primary endpoint analysis. The Wilcoxon rank sum test was used to compare groups on the longest seizure-free interval; the Hodges-Lehman estimator was used to calculate 95% CIs on the median difference between groups.

### Additional secondary endpoints

Each ZX008 dose group was compared with placebo on the proportion of patients who experienced ≥25% and ≥75% reduction in MCSF using logistic regression as described above. The Clinical Global Impression of Improvement (CGI-I) was assessed by the investigator and by the parent/caregiver on a 7-point Likert-like scale with responses ranging from "very much improved" to "no change" to "very much worse" this assessment was made during the blinded testing phase as well as during an open label extension (OLE) study having a treatment period of up to 24-months duration The proportion of subjects who were rated as "very much improved" or "much improved" in each fenfluramine dose group was compared to placebo using the Cochran-Mantel-Haenszel test stratified by age group.

The proportion of patients in each ZX008 dose group who experienced seizure freedom or near seizure freedom, defined as having ≤1 seizures, during the entire Treatment Period was compared to placebo.

All subjects received ZX008 or matching placebo for up to approximately 16 weeks (Titration Period=2 weeks; Maintenance Period=12 weeks; Taper/Transition Period=2 weeks) in Study 1. After completion of the Maintenance Period, eligible subjects could enroll in an open-label extension (OLE) study, after completion of the transition. Subjects who did not enroll in the OLE study were tapered off of study medication (doses were administered in a blinded fashion similar to the titration, i.e., doses were decreased in 4-day increments). Follow-up cardiovascular safety assessments, including ECG and ECHO, were performed 3 to 6 months following the last dose of study medication.

In Study 1, subjects were assessed using Clinical Global Impression (CGI), allowing the parent/caregiver and principal investigator to assess improvement, as well as the Quality of Life in Childhood Epilepsy (QOLCE) Scale and the Pediatric Quality of Life Inventory (PedsQL) to measure changes in the quality of life of the subject. Comparisons between treatment groups were made using Wilcoxon rank sum tests.

Missing data were not imputed for analysis of efficacy endpoints.

The CGI scale measures the change in the subject's clinical status from a specific point in time, i.e., the Baseline Period (and also referred to as the CGI-S, an initial ranking of severity). The CGI rating scale permits a global evaluation of the subject's improvement over time. The severity of a patient's condition is rated on a 7-point scale ranging from 1 (very much improved) to 7 (very much worse) as follows:
1=very much improved
2=much improved
3=minimally improved
4= no change
5=minimally worse
6=much worse
7=very much worse

In 2003, Varni and colleagues published a study intended to determine the feasibility, reliability, and validity of the 23-item PEDSQL 4.0 (Pediatric Quality of Life Inventory) Generic Core Scales as a measure of pediatric population health for children and adolescents. The instrument was given to over 10,000 families with children age 2-16 in the state of California. From Varni, *et al.,* parent reported changes in each of the scales and summary scores that could be considered clinically meaningful include a change of > 1 SEM or Standard Error of Measurement as a cut point.

In both active treatment groups experienced significant improvements in quality of life based on the PEDSQL total score. Both active treatment groups experienced greater improvement on all four core and three summary scores vs. placebo.

Scores in the active treatment groups observed to be improved as compared to scores in the placebo groups indicated that fenfluramine had an effect on the cognitive function(s) measured using the BRIEF scale.

### Results

A total of 173 patients were screened for eligibility and 119 patients enrolled and were randomized to treatment. There were 54 screening failures, the two most common reasons for screen failure were the presence of cardiovascular or cardiopulmonary abnormality (primarily trace mitral and/or trace aortic valve regurgitation (n=23, 43%) and failure to meet randomization requirements (n=19, 35%). Nine patients withdrew before completion of the study, three in the placebo group (lack of efficacy (n=1), subject/guardian decision (n=2)) and six in the ZX008 0.8 mg/kg/day group (adverse event (n=5), subject/guardian decision (n=1)). A total of 112 patients from Study 1 entered the OLE study.

Patient demographics and baseline seizure frequency are presented in Table 1. The average age of patients was 9.0±4.7 years and the mean MCSF was 41.9±65. Patients were currently being treated with a mean 2.4±1.0 (median, 2; range, 0 to 5). AEDs which most commonly included valproate (59.7%), clobazam (58.8%), topiramate (25.2%) and levetiracetam (21.8%). Fifty-eight (48.7%) subjects had previously been treated with stiripentol. Nonconvulsive seizures were reported in 24 patients in the ZX008 0.8 mg/kg/day group (60%), 23 patients in the ZX008 0.2 mg/kg/day group (59%), and 21 patients in the placebo group (53%). Overall mean compliance to study medication was >90% in each treatment group.

### Seizure Frequency

The study met its primary efficacy endpoint with high statistically significance. Compared with placebo, the ZX008 0.8 mg/kg/day group demonstrated a 63.9% greater reduction in mean MCSF over the 14-week treatment period (P<0.001, Table 2). The ZX008 0.2 mg/kg/day group also demonstrated a significant 33.7% greater reduction in mean MCSF compared with placebo (P=0.019, Table 2). Clinically meaningful reductions from baseline in mean MCSF were recorded at all timepoints measured during the Maintenance Period for the 0.8 mg/kg/day group: -62.4%, -61.8%, and -62.2% after 6, 10, and 14 weeks of treatment, respectively. Smaller, equally well sustained, reductions were observed in the 0.2 mg/kg/day group: -26.3%, -22.4%, and -22.2% at the same timepoints. A significantly greater proportion of subjects treated with either dose of ZX008 demonstrated a clinically meaningful (≥50%) or a substantial reduction (≥75%) in MCSF during the Treatment Period compared with subjects in the placebo group (Table 2), The median longest seizure-free interval was significantly longer in the ZX008 0.8 mg/kg/day group (20.5 days; p < 0.001) and ZX008 0.2 mg/kg/day group (14 days; p=0.011) compared with placebo (9 days; Table 2). Seizure freedom during the entire 14-week treatment period was experienced by 3 (7.5%) subjects in the ZX008 0.8 mg/kg/day group, 3 (7.7%) of subjects in the ZX008 0.2 mg/kg/kg group, and no subjects in the placebo group.

Given the high rate of seizures in Dravet Syndrome patients a post-hoc analysis was conducted to explore treatment effect on achieving a state of near-seizure freedom which was defined as experiencing 0 or only a single seizure during the 14-week Treatment Period. Near-seizure freedom was demonstrated in 10 (25%) subjects in the ZX008 0.8 mg/kg/day group, 5 (12.8%) subjects in the ZX008 0.2 mg/kg/day group, and 0 subjects in the placebo group.

### Quality of Life

Subjects in both active treatment groups experienced significant improvements in quality of life based on the Pediatric Quality of Life Inventory (PEDS-QL) total score (Table 2); however, no significant changes were seen in QOLCE.

### Safety

TEAEs were reported in 65% of the subjects in the placebo group and by 95% of subjects in each ZX008 dose group. A summary of non-cardiovascular adverse events that occurred in ≥10% of patients in any treatment group is presented in Table 3. The most common non-cardiovascular adverse events reported in ZX008-treated patients were decreased appetite, diarrhea, nasopharyngitis, lethargy, somnolence, and pyrexia. Among patients that had TEAEs, 93% were mild to moderate in severity; 24 (92.3%), 35 (94.6%), and 35 (92.1%) of patients in the placebo, 0.2 mg/kg/day, and 0.8 mg/kg/day groups, respectively.

Because fenfluramine had been marketed at higher doses as an anorectic drug, body weight was monitored throughout the trial and any change from baseline ≥7% was considered clinically meaningful. Overall, in the placebo group, 1 (2.5%) subject lost weight (maximum 8.0% at Visit 8) and 9 (22.5%) subjects had increases in weight that ranged from 7.4% to 17.1%, which was expected in a pediatric trial. In the ZX008 0.2 mg/kg/day group, 5 (12.8%) subjects had weight losses ranging from 8.4% to 21.9% of body weight; the one subject with a 21.9% loss was under the guidance of a nutritionist to manage her weight during the trial, and another subject with a 15.3% loss had been diagnosed with diabetes mellitus shortly before enrolling in the study and was also being managed to lose weight. One subject in the 0.2 mg/kg/day group gained 10.3% body weight during the study. In the ZX008 0.8 mg/kg/day group, 8 (20.0%) subjects lost weight, ranging from 7.2% to 11.4% of baseline body weight. One subject in the ZX008 0.8 mg/kg/day group discontinued citing decreased appetite and weight loss, among other events. The weight loss, however, amounted to less than 1 kg. There were no deaths in the study.

Serious adverse events occurred in 4 (10.0%) subjects in the placebo group, 4 (10.3%) subjects in the ZX008 0.2 mg/kg/day group, and in 5 (12.5%) subjects in the ZX0080.8 mg/kg/day group. These adverse events were primarily hospitalizations related to the disease under study, including status epilepticus in 2 (5.0%) placebo subjects, 1 (2.6%) ZX008 0.2 mg/kg/day subject, and 2 (5%) ZX008 0.8 mg/kg/day patients.

Compared to baseline, subjects randomized to ZX008 0.8mg/kg/day achieved statistically significant improvement on both the BRIEF Behavioral Regulation Index (BRI) (P < 0.05) and Global Executive Composite Score (GEC) (P < 0.05) as compared to placebo and subjects in the ZX008 0.2 mg/kg/day group achieved significant improvement on the BRI (P<0.05; Table 2). The BRI represents a child's ability to shift cognitive set and modulate emotions and behavior via appropriate inhibitory control; while GEC is a summary score that incorporates all eight clinical scales of the BRIEF. No changes in BRIEF-P were seen.

During Study 1, 5 (12.5%), 7 (17.9%), and 9 (22.5%) subjects in the placebo, ZX008 0.2 mg/kg/day, and ZX008 0.8 mg/kg/day groups, respectively, had at least one echocardiographic finding with trace mitral and/or trace aortic regurgitation. No cases of clinical or FDA-defined cardiac valvulopathy (mild or greater aortic regurgitation or moderate or greater mitral regurgitation) or pulmonary hypertension were observed. No clinically significant signs or symptoms characteristic of cardiovascular disease were seen at any time.

### Discussion

Dravet Syndrome is a severe refractory disabling childhood-onset epilepsy condition characterized by a high seizure burden accompanied by significant comorbid neurodevelopmental, motor, and behavioral abnormalities. In addition, the syndrome is marked by high mortality, most frequently due to status epilepticus and SUDEP. A Dravet-specific SUDEP rate of 9.32 per 1000-person years has been reported which is substantially higher than that reported in the general population of patients with epilepsy.

In this study ZX008 demonstrated a highly statistically significant and clinically meaningful dose-related reduction in the frequency of convulsive seizures with an early onset and a sustained effect. The significantly higher responder rates compared with placebo, particularly of patients demonstrating a ≥50% and ≥75% reductions in the frequency of convulsive seizures, further supports the robust anti-seizure efficacy of ZX008 in subjects with Dravet syndrome. These effects were assessed as clinically important by both the investigators and the parent/caregivers who rated a significantly larger proportion of ZX008-treated subjects as being "much improved" or "very much improved" compared with subjects in the placebo group.

Neurological comorbidities, including developmental delay, cognitive impairment, and behavioral issues, among others, are common in patients with Dravet syndrome, and current literature supports the concept that seizure frequency may be, at least in part, associated with the magnitude of these neurological deficits. In the present study, ZX008 was associated with a profound reduction in mean MCSF, with 45% of subjects in the 0.8 mg/kg/day group experiencing a ≥75% reduction. In this study significant improvements on some measures of quality of life began to emerge. The BRIEF was included as a safety measure to assess if treatment resulted in any negative effects on cognitive function. The results showed this was not the case; rather, statistically significant improvements in some of the executive function index scores on BRIEF were noted while the placebo group worsened on all three indexes. There is a hypothesis that the magnitude of reduction in MCSF may contribute to improvement in cognitive function. This concept is supported by a report from Catarino et al. who performed a retrospective study of a cohort of 22 adult patients with Dravet syndrome and found three of the patients who had experienced improvement in seizure control after being switched to appropriate AED as well as improvements in cognitive function. Further analyses of the full Phase 3 patient population, including the long term longitudinal assessment from the safety extension study is warranted to fully characterize treatment impact on quality of life and executive function.

ZX008 was generally well tolerated in this study. The safety profile of ZX008 with respect to non-cardiovascular events was similar to what has been reported for fenfluramine from the Belgian cohorts, with lethargy and decreases in appetite being reported more commonly in the present study in patients treated with ZX008 than with placebo. Fenfluramine was previously marketed as an appetite suppressant and therefore it is not unexpected that 21%-38% of subjects in the active treatment groups experienced decreases in appetite, but meaningful weight loss was less frequently reported (13% and 20%, in the ZX008 0.2 and 0.8 mg/kg/day groups, respectively. Serious adverse events occurred with similar frequency across the treatment groups and were mostly related to DS symptoms.

Cardiovascular safety remains an important consideration in the use of ZX008 to treat patients with Dravet syndrome. Fenfluramine was marketed from the 1960's to the late 1990's as a weight loss treatment, typically at doses of 60 to 120 mg/day, and most often in off-label combination with phentermine, another anorectic drug. Cardiovascular safety concerns emerged in the 1990s when it was reported that fenfluramine use was associated with an infrequent increased risk of primary pulmonary hypertension and cardiac valvulopathy. Based on these reports, fenfluramine was withdrawn from world-wide markets beginning in 1997. Estimating the magnitude of the risk of valvulopathy associated with fenfluramine has been complicated by the fact that pretreatment prevalence of cardiac valve disease in the obese adult patient population was not known and that phentermine was often used in combination with fenfluramine. A recent review of nine controlled studies of fenfluramine and related drugs in adults treated for obesity reported that the prevalence of mild or greater aortic regurgitation was 9.6% compared with 3.9% in the control groups, and the prevalence of moderate or greater mitral regurgitation was 3.1% compared with 2.5% in control groups. Higher doses of fenfluramine confer elevated risk for valve dysfunction as reported by Li et al. who analyzed the original cases referred to the FDA and stated that the relative risk of severe valvulopathy was significantly greater in obese adults taking ≥60 mg/day than in those taking <40 mg/day.

In the present study, all subjects were treated with ≤ 30 mg/day of ZX008 and were carefully monitored on a regular basis to identify functional changes in cardiac valves. During the 14-week treatment period and the 2-week transition period at the end of the Maintenance Period, no cases of cardiac valvulopathy or pulmonary hypertension were observed in any subject at any time. Only trace mitral and/or trace aortic regurgitation were seen, which are commonly seen in the general population and which are not recognized by current guidelines as an abnormality. Although the prevalence of trace regurgitation in young patients with Dravet syndrome is not known, 23 of 173 (13.3%) patients who were screened for participation in the present study were excluded due to the presence of trace mitral regurgitation on screening ECHO examination.

**Table 1. Demographics and baseline convulsive seizure frequency.**

| | **Placebo** | **ZX008 0.2 mg/kg/day** | **ZX008 0.8 mg/kg/day** | **Overall** |
|---|---|---|---|---|
| n | 40 | 39 | 40 | 119 |
| Age, years, mean±SD (min, max) | 9.2±5.1 (2, 18) | 9.0±4.5 (2, 17) | 8.8±4.4 (2, 18) | 9.0±4.7 (2, 18) |
| Age group <6 years, n (%) | 11 (27.5) | 9 (23.1) | 11 (27.5) | 31 (26.1) |
| Males, n (%) | 21 (52.5) | 22 (56.4) | 21 (52.5) | 64 (53.8) |

| Race, n (%) | | | | |
|---|---|---|---|---|
| Caucasian | 31 (77.5) | 33 (84.6) | 34 (85.0) | 98 (82.4) |
| Asian | 4 (10.0) | 2 (5.1) | 1 (2.5) | 7 (5.9) |
| Other or not reported* | 5 (12.5%) | 4 (10.3) | 5 (12.5) | 14 (11.8) |
| BMI, kg/m², mean±SD | 18.0±3.8 | 19.3±5.7 | 18.5±3.5 | 18.6± 4.4 |

| Geographic region, n (%) | | | | |
|---|---|---|---|---|
| United States | 23 (57.5) | 24 (61.5) | 23 (57.5) | 70 (58.8) |
| Canada | 1 (2.5) | 0 | 1 (2.5) | 2 (1.7) |
| Rest of world | 16 (40) | 15 (38.5) | 16 (40) | 47 (39.5) |
| Number of concomitant antiepileptic drugs mean±SD | 2.5±0.9 | 2.5±1.1 | 2.3±0.9 | 2.4±1.0 |

| Antiepileptic drugs, n (%) | | | | |
|---|---|---|---|---|
| Valproate (all forms) | 22 (55.0%) | 24 (61.5%) | 25 (62.5%) | 71 (59.8%) |
| Clobazam | 22 (55.0%) | 24 (61.5%) | 24 (60%) | 70 (58.8%) |
| Topiramate | 9 (22.5%) | 10 (25%) | 11 (27.5%) | 30 (25.2%) |
| Levetiracetam | 11 (27.5%) | 11 (28.2%) | 4 (10.0%) | 26 (21.8%) |
| Baseline convulsive seizure frequency per 28 days, mean±SD (median) | 46.1±40.7 (31.4) | 47.2±99.6 (17.5) | 33.0±31.5 (21.2) | 41.9±65.0 (22.7) |

**Table 2. Analysis of secondary endpoints**

| **Endpoint** | | | **Placebo** | **ZX008 0.2 mg/kg/day** | **ZX008 0.8 mg/kg/day** |
|---|---|---|---|---|---|
| | | | **n=40** | **n=39** | **n=40** |
| **Primary Endpoint:** | | | | | |
| Change in convulsive seizure frequency per 28 days | | | | | -63.89 (49.40, 74.22) |
| | % Difference from placebo, LS mean (95% CI) | | | | |
| | | | | | P<0.001 |

| **Secondary Endpoints** | | | | | |
|---|---|---|---|---|---|
| Change in convulsive seizure frequency per 28 days | | | | -33.74 (7.06, 52.77) | |
| | % Difference from placebo, LS mean endpoint(95% CI): key secondary | | | | |
| | | | | P=0.019 | |

| **Responder Analyses** | | | | | |
|---|---|---|---|---|---|
| ≥25 to reduction (n (%), OR [95% CI]) | | | 13 (32.5%) | 24 (61.5%) | 37 (92.5%) |
| | | | | 3.80 [1.44, 10.09] | 42.91 [9.39, 196.2] |
| | | | | P=0.007 | P<0.001 |
| ≥50% reduction: key secondary endpoint | | | 3 (7.5%) | 16 (41.0%) | 28 (70.0%) |
| | | | | 10.10 [2.48, 41.1] | 29.10 [7.18, 117.9] |
| | | | | P=0.001 | P<0.001 |
| ≥75% reduction | | | 1 (2.5%) | 8 (20.5%) | 18 (45.0%) |
| | | | | 10.46 [1.21, 90.52] | 49.49 [4.81, 509.1] P=0.001 |
| | | | | P=0.033 | |
| 100%^{a} | | | 0 | 3 (7.7%) | 3 (7.5%) |
| Longest seizure free interval: key secondary endpoint | | | 9 (2, 23) | 14 (3, 104) | 20.5 (2, 97) |
| | Days, median (range) | | | -5.0 [-9.0, -1.0] | -11.5 [-18.0, -5.0] |
| | Estimate difference of median treatment [95% CI] | | | P=0.011 | P<0.001 |
| Percent change in convulsive seizure frequency per 28 days | | | -17.4 (-76.1, 73.9) | -37.6 (-100, 220) | -72.4 (-100, 196.4) |
| | | Median (range) | -6.12±9.0 | -22.50±9.28 | -61.28±0.01 |
| | | LS means±SE | | P=0.185 | P<0.001 |

| **Non-seizure outcomes** | | | | | |
|---|---|---|---|---|---|
| **Endpoint** | | **Placebo** | **ZX008 0.2 mg/kg/day** | **ZX008 0.8 mg/kg/day** | |
| | | **n=40** | **n=39** | **n=40** | |
| **Clinical Global Impression of Improvement** | | | | | |
| Parent/caregiver rating, n (%) | | 4 (10.0%) | 16 (41.0%) | 22 (55.0%) | |
| | "very much improved" or "much improved" | | P=0.004 | P<0.001 | |
| | | 4 (10.0%) | 16 (41.0%) | 25 (55.0%) | |
| Investigator rating, n (%) | | | P=0.003 | P<0.001 | |
| | "very much improved" or "much improved" | | | | |

| **Quality of Life^{b}** | | | | | |
|---|---|---|---|---|---|
| **Pediatric Quality of Life Inventory (Peds QL)** | | | | | |
| | Total Score^{c} | 45.6±17.1 | 49.5±11.9 | 48.7±18.1 | |
| | Baseline±SD | -1.6±10.4 | 6.8±11.3 | 5.9±15.1 | |
| | Change from baseline, mean±SD | | P=0.003 | P=0.020 | |

| **Executive Function** | | | | | |
|---|---|---|---|---|---|
| **Behavioral Rating Inventory of Executive Function (BRIEF)**^{b,d} **Behavior Regulation Index (BRI)** | | 73.7±18.1 | 74.4±16.4 | 75.1±18.3 | |
| | | 3.0±8.7 (-0.54, 6.62) | -3.4±8.6 (-6.82, 0.01) | -4.4±10.5 (-8.34, - 0.52) | |
| | Baseline, mean ± SD | | | | |
| | Change from baseline, mean±SD (95% CI) | | P=0.018 | P=0.012 | |

| **Metacognition Index (MI)** | | | | | |
|---|---|---|---|---|---|
| | Baseline, mean ± SD | 103.7±25.1 | 104.0±23.9 | 106.3±25.0 | |
| | Change from baseline, mean±SD (95% CI) | 5.9±19.1 (-2.02,13.78) | -1.0±16.4 (-7.51, 5.44) | -6.6±20.7 (-14.32, 1.12) | |
| | | | P=0.199 | P=0.092 | |

| **Global Executive Composite** | | | | | |
|---|---|---|---|---|---|
| | Baseline, mean ± SD | 177.4±40.2 | 178.4±37.7 | 181.4±40.9 | |
| | Change from baseline, mean (95% CI) | 8.9±24.9 (-1.35, 19.19) | -4.4±22.3 (-13.27, 4.38) | -11.0±29.1 (-21.91, -0.15) | |
| | | | P=0.067 | P=0.025 | |

| | | | | | |
|---|---|---|---|---|---|
| ^{a} Because of the small number of subjects demonstrating 100% reduction in seizure frequency, model statistics such as the odds ratio are not reported ^{b} Results of QOLCE did not show statistically significant changes from placebo ^{c} Increases in total score indicates improvement ^{d} BRIEF was a safety endpoint ^{e} Because some countries do not have normative populations for BRIEF, only raw scores are presented here. ^{f}Negative scores indicate an improvement ^{g}Results of BRIEF-P revealed no statistical differences from placebo | | | | | |

**Table 1: Clinical Global Impression of Improvement, Parent/Caregiver Rating at Baseline (start of study 1503) and Final Visit (mITT Population)**

| **Summary Description** | **ZX008 OL (N=255)** |
|---|---|
| **OLE Baseline** | |
| Summary Statistics | |
| N | 225 |
| Mean | 3.1 |
| SE | 0.10 |
| Median | 3.0 |
| Min, Max | 1,7 |

| Number (%) of subjects responding | |
|---|---|
| 1= Very much improved | 37 (16.4) |
| 2= Much improved | 49 (21.8) |
| 3= Minimally improved | 43 (19.1) |
| 4= No change | 60 (26.7) |
| 5= Minimally worse | 24 (10.7) |
| 6= Much worse | 9 (4.0) |
| 7= Very much worse | 3 (1.3) |
| Much/very much improved (1, 2), n (%) | 86 (38.2) |
| 95% CI^{a} | (26.7, 51.4) |
| Improved (1, 2, 3) | 129 (57.3) |
| 95% CI^{a} | (42.5, 67.7) |

| **OLE Final Visit** | |
|---|---|
| Summary Statistics | |
| n | 248 |
| Mean | 2.4 |
| SE | 0.09 |
| Median | 2.0 |
| Min, Max | 1, 7 |
| Number (%) of subjects responding | |

| **Summary Description** | **ZX008 OL (N=255)** |
|---|---|
| 1= Very much improved | 67 (27.0) |
| 2= Much improved | 88 (35.5) |
| 3= Minimally improved | 49 (19.8) |
| 4= No change | 19 (7.7) |
| 5= Minimally worse | 13 (5.2) |
| 6= Much worse | 10 (4.0) |
| 7= Very much worse | 2 (0.8) |
| Much/very much improved (1, 2), n (%) | 155 (62.5) |
| 95% CI ^{a} | (52.5, 75.8) |
| Improved (1, 2, 3) | 204 (82.3) |
| 95% CI ^{a} | (72.3, 91.0) |

| | |
|---|---|
| Abbreviations: CGI = clinical global impression; CI = confidence interval; Max = maximum; mITT = modified intent-to-treat; Min = minimum; OL = open-label; SE: standard error. ^{a} Exact Clopper-Pearson 2-sided CI for the percentage of subjects with that response. | |

**Table 2: Clinical Global Impression of Improvement, Investigator Rating at Baseline (start of OLE), and Final Visit (mITT Population)**

| **Summary Description** | **ZX008 OL (N=255)** |
|---|---|
| **OLE Baseline** | |
| Summary Statistics | |
| N | 221 |
| Mean | 2.9 |
| SE | 0.08 |
| Median | 3.0 |
| Min, Max | 1,6 |

| N (%) of subjects responding | |
|---|---|
| 1= Very much improved | 35 (15.8) |
| 2= Much improved | 54 (24.4) |
| 3= Minimally improved | 46 (20.8) |
| 4= No change | 72 (32.6) |
| 5= Minimally worse | 11 (5.0) |
| 6= Much worse | 3 (1.4) |

| **Summary Description** | **ZX008 OL (N=255)** |
|---|---|
| 7= Very much worse | 0 (0.0) |
| Much/very much improved (1, 2), n (%) | 89 (40.3) |
| 95% CI ^{a} | (29.0, 54.4) |
| Improved (1, 2, 3) | 135 (61.1) |
| 95% CI^{a} | (42.5, 68.1) |

| **OLE Final Visit** | |
|---|---|
| Summary Statistics | |
| n | 254 |
| Mean | 2.3 |
| SE | 0.07 |
| Median | 2.0 |
| Min, Max | 1, 6 |

| N (%) of subjects responding | |
|---|---|
| 1= Very much improved | 70 (27.6) |
| 2= Much improved | 93 (36.6) |
| 3= Minimally improved | 54 (21.3) |
| 4= No change | 20 (7.9) |
| 5= Minimally worse | 14 (5.5) |
| 6= Much worse | 3 (1.2) |
| 7= Very much worse | 0 (0.0) |
| Much/very much improved (1, 2), n (%) | 163 (64.2) |
| 95% CI^{a} | (61.4, 83.1) |
| Improved (1, 2, 3) | 217 (85.4) |
| 95% CI ^{a} | (72.3, 91.0) |

| | |
|---|---|
| Abbreviations: CGI = clinical global impression; CI = confidence interval; Max = maximum; mITT = modified intent-to-treat; Min = minimum; OL = open-label; SE: standard error. ^{a} Exact Clopper-Pearson 2-sided CI for the percentage of subjects with that response. | |

### EXAMPLE 3

### THERAPY WITH FENFLURAMINE AND TESTING FOR IMPROVED COGNITIVE FUNCTION AS ASSESSED BY BRIEF - LENNOX-GASTAUT SYNDROME (LGS)

In this two-part study of fenfluramine HCl in children and adults with LGS, Part 1 is a randomized, double-blind, placebo-controlled trial of two fixed doses of fenfluramine HCl oral solution as adjunctive therapy for seizures in children and adults with LGS; Part 2 is an open label.
extension to assess long-term safety of ZX008 in children and adults with LGS.

In this study conducted in LGS patients, the BRIEF is administered study day 1, visit 15.

ZX008 drug product is an oral aqueous solution of fenfluramine hydrochloride buffered to pH 5 and provided in concentrations of 1.25 mg/mL, 2.5 mg/mL, and 5 mg/mL. The excipients selected have been approved for use in the formulations of currently marketed drug products and are considered to be safe. The solution formulations will be suitably flavored, and will contain preservatives and a thickening agent. The product is sugar free and is intended to be compatible with a KD.

The formulation for Part 1 will be provided in bottles with tamper-evident, child-resistantcaps. The clinical trials material will be supplied in 1 bottle size with nominal fill volume of120 mL. Matching placebo also will be provided. Doses to be studied include ZX008 0.2mg/kg/day and ZX008 0.8 mg/kg/day divided into two daily (BID) doses, up to a maximum of 30 mg/day (subjects taking concomitant STP will receive 0.2 mg/kg/day or 0.5 mg/kg/day, up to a maximum of 20 mg/kg/day). An intermediate dose of 0.4 mg/kg/day will be used for titration. The concentration of ZX008 oral solution received by subjects (1.25 mg/mL, 2.5mg/mL, and/or 5 mg/mL) will be randomized across the 3 available concentrations in order to ensure blinding.

For Part 2, the doses to be studied include 0.2 mg/kg/day, 0.4 mg/kg/day, 0.6 mg/kg/day, and 0.8 mg/kg/day divided into two daily doses, up to a maximum of 30 mg/day (subjects taking concomitant STP will receive 0.2 mg/kg/day, 0.4 mg/kg/day, or 0.5 mg/kg/day, up to a maximum of 20 mg/kg/day). ZX008 drug product will be provided in a concentration of 2.5mg/mL in 1 bottle size with nominal fill volume of 120 mL.

The preceding merely illustrates the principles of the invention. It will be appreciated that those skilled in the art will be able to devise various arrangements which, although not explicitly described or shown herein, embody the principles of the invention Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. The scope of the present invention, therefore, is not intended to be limited to the exemplary embodiments shown and described herein. Rather, the scope of present invention is embodied by the appended claims

## Claims

1. A formulation for use in a method of improving cognitive function in a patient with Dravet syndrome or Lennox-Gastaut syndrome, wherein the formulation comprises a therapeutically effective dose of fenfluramine or a pharmaceutically acceptable salt thereof.

2. The formulation for use of claim 1, wherein the use further comprises the administration of a co-therapeutic agent selected from the group consisting of, Brivaracetam, bromides (e.g., Potassium Bromide, Sodium Bromide), Cannabidiol, Carbamazepine, Clonidine, Ergenyl Chrono, Ethosuximide, Felbamate, Fosphenytoin, Lacosamide, Lamotrigine, Levetiracetam, Levocarnitine, Mesuximide, Nitrazepam, Oxcarbazepine, Perampanel, Phenobarbital, Pregabalin, Progabide, Pyridoxine, Rufinamide, Sultiame, Tizanidine, Topiramate, Stiripentol, Valproate semisodium, Valproate sodium, Valproic acid, Verapamil, Zonisamide, and benzodiazepines such as Clobazam, Clonazepam, Diazepam, Ethyl Loflazepate, Lorazepam and Midazolam, and a pharmaceutically acceptable salt or base thereof.

3. The formulation for use of claim 1 or 2, wherein the improvement in cognitive function is assessed using the Behavior Rating Inventory of Executive Function (BRIEF) test or the Clinical Global Impression (CGI) scale.

4. The formulation for use of any preceding claim, wherein the patient is a patient with Dravet syndrome.

5. The formulation for use of any preceding claim, wherein the patient is a patient with Lennox-Gastaut syndrome.

6. The formulation for use of any preceding claim, wherein the improvement in cognitive function is assessed using the Behavior Rating Inventory of Executive Function (BRIEF) test.

7. The formulation for use of any preceding claim, wherein the improvement in cognitive function is assessed using the Clinical Global Impression (CGI) scale.

8. The formulation for use of any preceding claim, wherein the fenfluramine is formulated with a pharmaceutically acceptable carrier, and administered in an effective dose of less than 1.0 mg/kg/day.

9. The formulation for use of any preceding claim, wherein the fenfluramine is formulated with a pharmaceutically acceptable carrier and administered in an effective dose selected from 0.8 mg/kg/day, 0.5 mg/kg/day, and 0.2 mg/kg/day.

10. The formulation for use of any preceding claim, wherein the fenfluramine is administered in an oral composition.

11. The formulation for use of any preceding claim, wherein the fenfluramine is administered in an amount of 30 mg/day or less.

12. The formulation for use of any preceding claim, wherein the fenfluramine is administered in an effective dose of 0.8 mg/kg/day.

13. The formulation for use of claim 1, wherein the fenfluramine is administered in an effective dose selected from 0.2 mg/kg/day, 0.4 mg/kg/day, and 0.7 mg/kg/day.

14. The formulation for use of claim 1, wherein the fenfluramine is administered in an effective dose of 0.7 mg/kg/day

## Patentansprüche

1. Formulierung zur Verwendung bei einem Verfahren zum Verbessern der kognitiven Funktion eines Patienten mit Dravet-Syndrom oder Lennox-Gastaut-Syndrom, wobei die Formulierung eine therapeutisch wirksame Dosis Fenfluramin oder eines pharmazeutisch annehmbaren Salzes davon umfasst.

2. Formulierung zur Verwendung nach Anspruch 1, wobei die Verwendung ferner die Verabreichung eines cotherapeutischen Mittels ausgewählt aus der Gruppe bestehend aus Brivaracetam, Bromiden (z.B. Kaliumbromid, Natriumbromid), Cannabidiol, Carbamazepin, Clonidin, Ergenyl Chrono, Ethosuximid, Felbamat, Fosphenytoin, Lacosamid, Lamotrigin, Levetiracetam, Levocarnitin, Mesuximid, Nitrazepam, Oxcarbazepin, Perampanel, Phenobarbital, Pregabalin, Progabid, Pyridoxin, Rufinamid, Sultiame, Tizanidin, Topiramat, Stiripentol, Valproat-Seminatrium, Valproat-Natrium, Valproinsäure, Verapamil, Zonisamid und Benzodiazepine, wie z.B. Clobazam, Clonazepam, Diazepam, Ethylloflazepat, Lorazepam und Midazolam, und einem pharmazeutisch annehmbaren Salz oder einer pharmazeutisch annehmbaren Base davon umfasst.

3. Formulierung zur Verwendung nach Anspruch 1 oder 2, wobei die Verbesserung der kognitiven Funktion unter Verwendung des Behavior Rating Inventory of Executive Function (BRIEF)-Tests oder der Clinical Global Impression (CGI)-Skala bewertet wird.

4. Formulierung zur Verwendung nach einem der vorstehenden Ansprüche, wobei der Patient ein Patient mit Dravet-Syndrom ist.

5. Formulierung zur Verwendung nach einem der vorstehenden Ansprüche, wobei der Patient ein Patient mit Lennox-Gastaut-Syndrom ist.

6. Formulierung zur Verwendung nach einem der vorstehenden Ansprüche, wobei die Verbesserung der kognitiven Funktion unter Verwendung des Behavior Rating Inventory of Executive Function (BRIEF)-Tests bewertet wird.

7. Formulierung zur Verwendung nach einem der vorstehenden Ansprüche, wobei die Verbesserung der kognitiven Funktion unter Verwendung der Clinical Global Impression (CGI)-Skala bewertet wird.

8. Formulierung zur Verwendung nach einem der vorstehenden Ansprüche, wobei das Fenfluramin mit einem pharmazeutisch verträglichen Träger formuliert und in einer wirksamen Dosis von weniger als 1,0 mg/kg/Tag verabreicht wird.

9. Formulierung zur Verwendung nach einem der vorstehenden Ansprüche, wobei das Fenfluramin mit einem pharmazeutisch verträglichen Träger formuliert und in einer wirksamen Dosis ausgewählt aus 0,8 mg/kg/Tag, 0,5 mg/kg/Tag und 0,2 mg/kg/Tag verabreicht wird.

10. Formulierung zur Verwendung nach einem der vorstehenden Ansprüche, wobei das Fenfluramin in einer oralen Zusammensetzung verabreicht wird.

11. Formulierung zur Verwendung nach einem der vorstehenden Ansprüche, wobei das Fenfluramin in einer Menge von 30 mg/Tag oder weniger verabreicht wird.

12. Formulierung zur Verwendung nach einem der vorstehenden Ansprüche, wobei das Fenfluramin in einer wirksamen Dosis von 0,8 mg/kg/Tag verabreicht wird.

13. Formulierung zur Verwendung nach Anspruch 1, wobei das Fenfluramin in einer wirksamen Dosis ausgewählt aus 0,2 mg/kg/Tag, 0,4 mg/kg/Tag und 0,7 mg/kg/Tag verabreicht wird.

14. Formulierung zur Verwendung nach Anspruch 1, wobei das Fenfluramin in einer wirksamen Dosis von 0,7 mg/kg/Tag verabreicht wird.

## Revendications

1. Formulation pour une utilisation dans un procédé d'amélioration de la fonction cognitive chez un patient atteint du syndrome de Dravet ou du syndrome de Lennox-Gastaut, la formulation comprenant une dose thérapeutiquement efficace de fenfluramine ou d'un sel pharmaceutiquement acceptable de celle-ci.

2. Formulation pour une utilisation de la revendication 1, l'utilisation comprenant en outre l'administration d'un co-agent thérapeutique choisi dans le groupe constitué par le brivaracétam, les bromures (par ex., bromure de potassium, bromure de sodium), le cannabidiol, la carbamazépine, la clonidine, l'Ergenyl Chrono, l'éthosuximide, le felbamate, la fosphénytoïne, le lacosamide, la lamotrigine, le lévétiracétam, la lévocarnitine, le mésuximide, le nitrazépam, l'oxcarbazépine, le pérampanel, le phénobarbital, la prégabaline, le progabide, la pyridoxine, le rufinamide, le sultiame, la tizanidine, le topiramate, le stiripentol, le valproate semi-sodique, le valproate sodique, l'acide valproïque, le vérapamil, le zonisamide, et les benzodiazépines telles que le clobazam, le lonazépam, le diazépam, le loflazépate d'éthyle, le lorazépam et le midazolam, et les sels ou bases pharmaceutiquement acceptables de ceux-ci.

3. Formulation pour une utilisation de la revendication 1 ou 2, l'amélioration de la fonction cognitive étant évaluée au moyen du test de l'inventaire d'évaluation comportementale des fonctions exécutives (BRIEF) ou de l'échelle d'impression clinique globale (CGI).

4. Formulation pour une utilisation d'une quelconque revendication précédente, le patient étant un patient atteint du syndrome de Dravet.

5. Formulation pour une utilisation d'une quelconque revendication précédente, le patient étant un patient atteint du syndrome de Lennox-Gastaut.

6. Formulation pour une utilisation d'une quelconque revendication précédente, l'amélioration de la fonction cognitive étant évaluée au moyen du test de l'inventaire d'évaluation comportementale des fonctions exécutives (BRIEF).

7. Formulation pour une utilisation d'une quelconque revendication précédente, l'amélioration de la fonction cognitive étant évaluée au moyen de l'échelle d'impression clinique globale (CGI).

8. Formulation pour une utilisation d'une quelconque revendication précédente, la fenfluramine étant formulée avec un véhicule pharmaceutiquement acceptable et administrée à une dose efficace inférieure à 1,0 mg/kg/jour.

9. Formulation pour une utilisation d'une quelconque revendication précédente, la fenfluramine étant formulée avec un véhicule pharmaceutiquement acceptable et administrée à une dose efficace choisie parmi 0,8 mg/kg/jour, 0,5 mg/kg/jour et 0,2 mg/kg/jour.

10. Formulation pour une utilisation d'une quelconque revendication précédente, la fenfluramine étant administrée dans une composition orale.

11. Formulation pour une utilisation d'une quelconque revendication précédente, la fenfluramine étant administrée dans une quantité de 30 mg/jour ou moins.

12. Formulation pour une utilisation d'une quelconque revendication précédente, la fenfluramine étant administrée à une dose efficace de 0,8 mg/kg/jour.

13. Formulation pour une utilisation de la revendication 1, la fenfluramine étant administrée à une dose efficace choisie parmi 0,2 mg/kg/jour, 0,4 mg/kg/jour et 0,7 mg/kg/jour.

14. Formulation pour une utilisation de la revendication 1, la fenfluramine étant administrée à une dose efficace de 0,7 mg/kg/jour.
